# EUROPEAN PATENT SPECIFICATION

(11) **EP 1 127 161 B1**
(45) Date of publication and mention of the grant of the patent: **01.02.2006**
(21) Application number: 99958733.0
(22) Date of filing: 01.11.1999
(51) Int. Cl.: C12Q 1/68, C07H 21/02

(54) **METHOD FOR MAKING COMPLEMENTARY OLIGONUCLEOTIDE TAG SETS**
METHODE ZUR HERSTELLUNG VON SÄTZEN KOMPLEMENTÄRER OLIGONUCLEOTIDE
TECHNIQUE PERMETTANT D'OBTENIR DES ENSEMBLES DE MARQUEURS OLIGONUCLEOTIDIQUES COMPLEMENTAIRES

(30) Priority: 02.11.1998 US 106662 P
(43) Date of publication of application: 29.08.2001
(73) Proprietor: Solexa, Inc, Hayward CA 94545 (US)
(72) Inventor: WILLIAMS, Steven, R., San Francisco, CA 94114 (US); KIRCHNER, James, J., Menlo Park, CA 94025 (US); DUBRIDGE, Robert, B., Belmont, CA 94002 (US)
(74) Representative: Vossius & Partner
(86) International application number: PCT/US1999/025680
(87) International publication number: WO 2000/026411

(56) References cited:
- WO-A-96/12014
- WO-A1-96/41011
- US-A- 5 763 175
- US-A- 5 780 231
- US-A- 5 962 228
- US-A- 6 013 445
- BRENNER S ET AL: "In vitro cloning of complex mixtures of DNA on microbeads: Physical separation of differentially expressed cDNAs" PROCEEDINGS OF THE NATIONAL ACADEMY OF SCIENCES OF USA, NATIONAL ACADEMY OF SCIENCE. WASHINGTON, US, vol. 97, no. 4, 15 February 2000 (2000-02-15), pages 1665-1670, XP002183229 ISSN: 0027-8424

### SEQUENCE LISTING

<110> Lynx Therapeutics, Inc.
<120> Method for Making Complementary Oligonucleotide Tag Sets
<130> F 1593 EP
<140> PCT/US99/25680
   <141> 1999-11-01
<150> US 60/106,662
   <151> 1998-11-02
<160> 27
<170> FastSEQ for Windows Version 4.0
<210> 1
   <211> 28
   <212> DNA
   <213> Artificial Sequence
<220>
   <223> primer
<400> 1
   tccttaatta actggtctca ctgtcgca 28
<210> 2
   <211> 39
   <212> DNA
   <213> Artificial Sequence
<220>
   <223> oligonucleotide
   <221> misc_feature
   <222> (1) ... (39)
   <223> n = SEQ ID 20, 21, 22, 23, 24, 25, 26, or 27
<400> 2
   cccnnnnnnn ntccttaatt aactggtctc actgtcgca 39
<210> 3
   <211> 8
   <212> DNA
   <213> Artificial Sequence
<220>
   <223> oligonucleotide
<400> 3
   ccttaggg 8
<210> 4
   <211> 47
   <212> DNA
   <213> Artificial Sequence
<220>
   <223> oligonucleotide
   <221> misc_feature
   <222> (1)...(47)
   <223> n = SEQ ID 20, 21, 22, 23, 24, 25, 26, or 27
<400> 4
   ccttagggcc cnnnnnnnnt ccttaattaa ctggtctcac tgtcgca 47
<210> 5
   <211> 74
   <212> DNA
   <213> Artificial Sequence
<220>
   <223> oligonucleotide
<400> 5
   cgagaaagag ggataaggct cgagcttaat taagagtcga cgaattcggg cccggatcct 60
   gactctttct ccct 74
<210> 6
   <211> 82
   <212> DNA
   <213> Artificial Sequence
<220>
   <223> oligonucleotide
<400> 6
   ctagagggag aaagagtcag gatccgggcc cgaattcgtc gactcttaat taagctcgag 60
   ccttatccct ctttctcggt ac 82
<210> 7
   <211> 47
   <212> DNA
   <213> Artificial Sequence
<220>
   <223> oligonucleotide
<400> 7
   tcgaggcata agtcttcgaa ttccatcaca ctgggaagac aacgtag 47
<210> 8
   <211> 47
   <212> DNA
   <213> Artificial Sequence
<220>
   <223> oligonucleotide
<400> 8
   gatcctacgt tgtcttccca gtgtgatgga attcgaagac ttatgcc 47
<210> 9
   <211> 73
   <212> DNA
   <213> Artificial Sequence
<220>
   <223> oligonucleotide
<400> 9
   tcgattaatt aacaagcttt gggccctcga gcataagtct tctgcagaat tcggatccat 60
   cgatggtcat agc 73
<210> 10
   <211> 45
   <212> DNA
   <213> Artificial Sequence
<220>
   <223> oligonucleotide
<400> 10
   tgtttcctgc cacacaacat acgagccgga agcggccgct ctaga 45
<210> 11
   <211> 61
   <212> DNA
   <213> Artificial Sequence
<220>
   <223> oligonucleotide
<400> 11
   agcgtctaga gcggccgctt ccggctcgta tgttgtgtgg caggaaacag ctatgaccat 60
   c 61
<210> 12
   <211> 57
   <212> DNA
   <213> Artificial Sequence
<220>
   <223> oligonucleotide
<400> 12
   gatggatccg aattctgcag aagacttatg ctcgagggcc caaagcttgt taattaa 57
<210> 13
   <211> 22
   <212> DNA
   <213> Artificial Sequence
<220>
   <223> oligonucleotide
<400> 13
   tcgagggccc gcataagtct tc 22
<210> 14
   <211> 22
   <212> DNA
   <213> Artificial Sequence
<220>
   <223> oligonucleotide
<400> 14
   tcgagaagac ttatgcgggc cc 22
<210> 15
   <211> 35
   <212> DNA
   <213> Artificial Sequence
<220>
   <223> plasmid
<400> 15
   ttaattaagg agggcccgca taagtcttcg gatcc 35
<210> 16
   <211> 16
   <212> DNA
   <213> Artificial Sequence
<220>
   <223> primer
<400> 16
   tgtggcttaa ttaagg 16
<210> 17
   <211> 42
   <212> DNA
   <213> Artificial Sequence
<220>
   <223> primer
<221> misc_feature
   <222> (1)...(42)
   <223> v = A, C or G
<400> 17
   gacatgctgc attgagacga ttcttttttt tttttttttt tv 42
<210> 18
   <211> 48
   <212> DNA
   <213> Artificial Sequence
<220>
   <223> oligonucleotide
<221> misc_feature
   <222> (1)...(48)
   <223> v = A, C, or G
<221> misc_feature
   <222> (1)...(48)
   <223> n = unknown number of nucleotides
<400> 18
   gacatgctgc attgagacga ttcttttttt tttttttttt tvngatcn 48
<210> 19
   <211> 39
   <212> DNA
   <213> Artificial Sequence
<220>
   <223> oligonucleotide
<221> misc_feature
   <222> (1) ... (39)
   <223> n = unknown number of nucleotides
<221> misc_feature
   <222> (1)...(39)
   <223> v = A, C, or G
<400> 19
   gcattgagac gattcttttt tttttttttt tttvctttn 39
<210> 20
   <211> 4
   <212> DNA
   <213> Artificial Sequence
<220>
   <223> oligonucleotide
<400> 20
   cttt 4
<210> 21
   <211> 4
   <212> DNA
   <213> Artificial Sequence
<220>
   <223> oligonucleotide
<400> 21
   tact 4
<210> 22
   <211> 4
   <212> DNA
   <213> Artificial Sequence
<220>
   <223> oligonucleotide
<400> 22
   acat 4
<210> 23
   <211> 4
   <212> DNA
   <213> Artificial Sequence
<220>
   <223> oligonucleotide
<400> 23
   aatc 4
<210> 24
   <211> 4
   <212> DNA
   <213> Artificial Sequence
<220>
   <223> oligonucleotide
<400> 24
   ttac 4
<210> 25
   <211> 4
   <212> DNA
   <213> Artificial Sequence
<220>
   <223> oligonucleotide
<400> 25
   tcta 4
<210> 26
   <211> 4
   <212> DNA
   <213> Artificial Sequence
<220>
   <223> oligonucleotide
<400> 26
   atca 4
<210> 27
   <211> 4
   <212> DNA
   <213> Artificial Sequence
<220>
   <223> oligonucleotide
<400> 27
   caaa 4

## Description

### Field of the Invention

The present invention relates generally to methods for synthesizing collections of oligonucleotide tags which may be used for identifying, sorting, and/or tracking molecules, especially polynucleotides. In a particular aspect, the present invention relates to a method for forming complementary sets of oligonucleotide tags and tag complements, which are particularly useful in immobilizing tagged entities on addressable arrays of tag complements.

### Background of the Invention

Specific hybridization of oligonucleotides and their analogs is a fundamental process that is employed in a wide variety of research, medical, and industrial applications, including the identification of disease-related polynucleotides in diagnostic assays, screening for clones of novel target polynucleotides, identification of specific polynucleotides in blots of mixtures of polynucleotides, amplification of specific target polynucleotides, therapeutic blocking of inappropriately expressed genes, DNA sequencing, and the like, e.g. Sambrook et al., Molecular Cloning: A Laboratory Manual, 2nd Edition (Cold Spring Harbor Laboratory, New York, 1989); Keller and Manak, DNA Probes, 2nd Edition (Stockton Press, New York, 1993); Milligan et al., J. Med. Chem., 36: 1923-1937 (1993); Drmanac et al., Science, 260: 1649-1652 (1993); Bains, J., DNA Sequencing and Mapping, 4: 143-150 (1993).

Specific hybridization has also been proposed as a method of tracking, retrieving, and identifying compounds labeled with oligonucleotide tags, e.g. Brenner (Pub. No. WO 96/12014); Church et al., Science, 240: 185-188 (1988); Brenner and Lemer, Proc. Natl. Acad. Sci., 89: 5381-5383 (1992); Alper, Science, 264: 1399-1401 (1994); and Needels et al., Proc. Natl. Acad. Sci., 90: 10700-10704 (1993). The successful implementation of such tagging schemes depends in large part on the success in achieving specific hybridization between a tag and its complementary probe. That is, for an oligonucleotide tag to successfully identify a substance, the number of false positive and false negative signals must be minimized. Unfortunately, such spurious signals are not uncommon because base pairing and base stacking free energies vary widely among nucleotides in a duplex or triplex structure. For example, a duplex consisting of a repeated sequence of deoxyadenosine (A) and thymidine (T) bound to its complement may have less stability than an equallength duplex consisting of a repeated sequence of deoxyguanosine (G) and deoxycytidine (C) bound to a partially complementary target containing a mismatch. Thus, if a desired compound from a large combinatorial chemical library were tagged with the former oligonucleotide, a significant possibility would exist that, under hybridization conditions designed to detect perfectly matched AT-rich duplexes, undesired compounds labeled with the GC-rich oligonucleotide, even in a mismatched duplex, would be detected along with the perfectly matched duplexes consisting of the AT-rich tag. Even though reagents, such as tetramethylammonium chloride, are available to negate base-specific stability differences of oligonucleotide duplexes, the effect of such reagents is often limited, and their presence can be incompatible with, or render more difficult, further manipulations of the selected compounds, e.g. amplification by polymerase chain reaction (PCR), or the like. Such problems have been addressed by Brenner, as described in WO 96/12014 (supra) and WO 96/41011 in the development of minimally cross-hybridizing oligonucleotide tag sets which minimize the occurrence of mismatch hybridization.

Unfortunately, however, current methods of solid phase oligonucleotide synthesis, although highly efficient, still result in a significant fraction of failure sequences, particularly when oligonucleotides exceed 30 to 40 nucleotides in length. When mixtures of such oligonucleotides are employed as tags, particularly for sorting, an important consequence of failure sequences is that a fraction of the tags will not have complements whenever the two compounds are synthesized separately. Thus, in the sorting of complex mixtures, e.g. as called for in the process described in Brenner (cited above), such failures will limit the completeness of the sorting process.

In view of the above, it would be useful if there were available a means for synthesizing sets of oligonucleotide tags that would ensure that every tag had a complement regardless of the efficiency of the synthetic process employed.

### Summary of the Invention

An object of the present invention is to provide a method of synthesizing one or more repertoires of oligonucleotide tags that ensures that every oligonucleotide tag in a repertoire will have a corresponding tag complement.

Another object of the invention is to provide a system for sorting polynucleotides onto solid phase supports by way of oligonucleotide tags produced by the method of the invention.

A further object of the invention is to provide a method of synthesizing oligonucleotide tags by the combinatorial addition of subunits (words).

These and other objects of the invention are achieved by providing a method of synthesis comprising the steps of synthesizing a repertoire of oligonucleotide tag complements on one or more solid phase supports, cleaving a fraction of the oligonucleotide tag complements from the support(s), and inserting the cleaved tag complements into a cloning vector. The cloned tag complements can conveniently be conjugated to selected substances, preferably biomolecules such as polynucleotides or polypeptides, to produce tagged substances having unique tag sequences which can be captured and sorted by hybridization to corresponding tag complements.

In one embodiment, the invention provides a method of synthesizing a repertoire of oligonucleotide tags. In the method, a plurality of different sequence oligonucleotide populations are synthesized on one or more solid phase supports, such that each population is located at a spatially discrete region relative to the other populations, and the oligonucleotides in each population comprise a tag-complement sequence that is the same for every oligonucleotide in a given population, but which may be different from the tag-complement sequence in every other oligonucleotide population. A fraction of the oligonucleotides are cleaved from each population on the one or more supports, to release a mixture of different-sequence tag-complement oligonucleotides. A primer is annealed to each tag-complement oligonucleotide, and the annealed primers are extended to form a duplex comprising a tag-complement strand and a complementary tag strand, such that the tag-complement strands or the duplexes thus formed comprise an oligonucleotide tag repertoire. In one preferred embodiment, the oligonucleotides are bound to the one or more supports by their 3'-termini, and each oligonucleotide contains a universal primer-binding sequence on the 3'-side of the tag complement sequence. In another preferred embodiment, the fraction of oligonucleotides cleaved from each population is from 10 to 30%. According to yet another preferred embodiment, a selected fraction of each population of synthesized oligonucleotides is bound to the one or more supports via base-cleavable linkages.

The disclosure also includes a repertoire of tag oligonucleotides prepared as above.

Also provided is a method of forming a tag-vector library comprising multiple members from a tag repertoire. In the method, an oligonucleotide tag repertoire prepared as above is inserted by ligation into multiple copies of a selected vector, to form a tag-vector library comprising members of the tag repertoire. The invention also includes a tag-vector library formed by the method.

The invention also includes a method of forming a library of tagged polynucleotide fragments. In the method, a tag-vector library from above is combined with a mixture of different polynucleotide fragments under ligation conditions effective to insert a polynucleotide fragment into each tag-vector at a site adjacent to a tag-sequence in each vector. The disclosure also includes a library of tagged polynucleotide fragments formed by the method.

In yet another aspect, the disclosure also includes a system for sorting polynucleotides. The system includes one or more solid phase supports on which are attached a plurality of oligonucleotide populations, such that each population is located at a spatially discrete region on the one or more supports relative to the other populations, and the oligonucleotides in each population comprise a tag-complement sequence that is the same for every oligonucleotide in a given population. The system also includes a tag composition selected from the group consisting of a tag repertoire, a tag-vector library, or a library of tagged polynucleotide fragments, which may be prepared as above.

The invention also provides a solid phase support comprising a plurality of immobilized oligonucleotides containing identical tag sequences, wherein at least one of the oligonucleotides is linked to the support by a cleavable linker, and at least one other of the oligonucleotides is linked to the support by a non-cleavable linker. In a first preferred embodiment, the support is a bead. In a second preferred embodiment, the support comprises a planar array of addressable regions, each region containing a plurality of immobilized oligonucleotides containing identical tag sequences, such that the tag sequences in at least two of the regions are different from each other. Preferably, the cleavable linker is a base-labile linker. The solid phase supports of the invention are particularly useful for generating repertoires of tag oligonucleotides that are substantially complementary to the oligonucleotides immobilized on the supports.

Thus, the invention also includes a solid phase bead or particle comprising a population of oligonucleotides attached to the bead or particle, wherein the population comprises first and second classes of oligonucleotides which contain identical oligonucleotide sequences, and the oligonucleotides in the first class contain a cleavable linking moiety that permits selective cleavage of the first class of oligonucleotides from the support, without significantly cleaving the second class of oligonucleotides.

In another embodiment, the invention includes a mixture of solid phase beads or particles of the type described above, wherein at least two beads or particles in the mixture contain oligonucleotide populations with different tag-complement sequences.

These and other objects and features of the present invention will become more apparent from the following description and the appended drawings.

### Brief Description of the Drawings

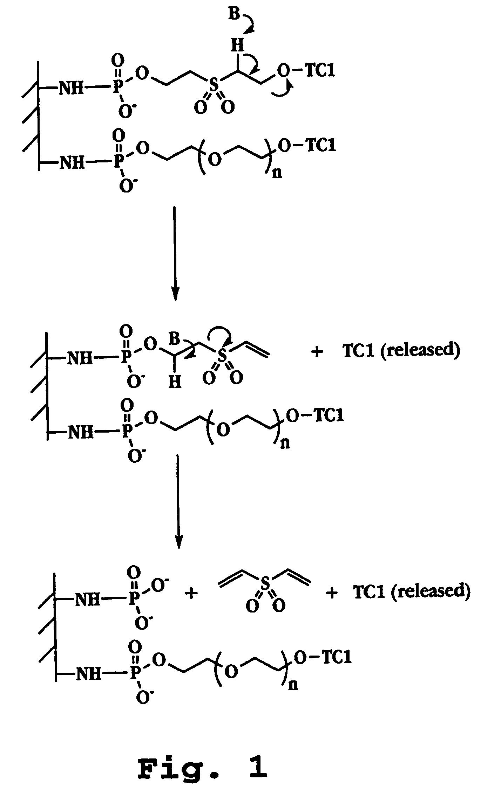

Figure 1 shows an exemplary pair of oligonucleotide linking structures bound to a solid phase bead, and a mechanism of cleaving one of the linkers to release an attached oligonucleotide containing a tag complement (TC 1), while the other linker remains intact.

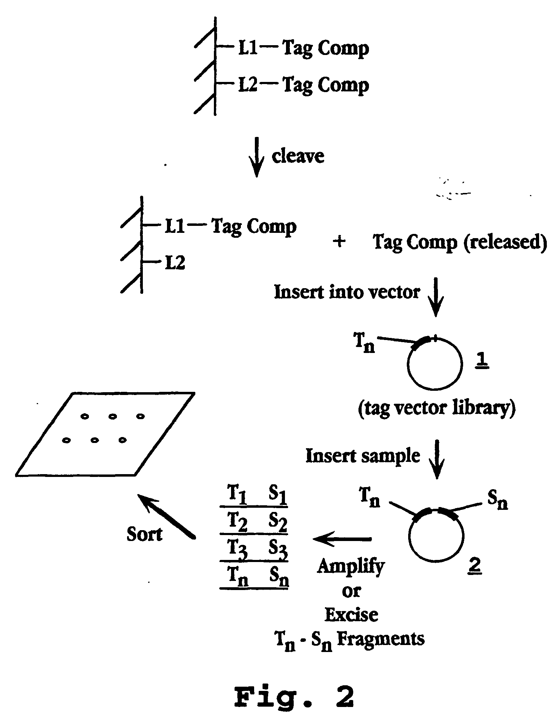

Figure 2 shows a general scheme for practicing one embodiment of the invention, wherein a portion of a repertoire of immobilized oligonucleotides containing different-sequence tag complements are cleaved from the solid phase support, converted to double-stranded form, and inserted into a cloning vector. A tag-vector (1) library is combined with a mixture of different polynucleotide fragments under ligation conditions effective to insert the different fragments at a site adjacent to a tag-sequence in each vector, so that each sample fragment (Sₙ) becomes associated with a different tag (Tₙ). The tag-sample fragments may then be excised and/or amplified, and the tagged fragments sorted by hybridization to immobilized corresponding tag complements.

### Detailed Description of the Invention

The present invention provides methods and reagents for synthesizing matched sets (also referred to as repertoires) of tags and tag complements having high complementarity with each other. Such matched sets are particularly useful for labeling and sorting tagged molecules for parallel operations such as sequencing, fingerprinting, or other types of analysis.

In one aspect of the invention, populations of oligonucleotides are synthesized wherein each population contains identical tag complements that are linked to a support by a mixture of cleavable and non-cleavable linking moieties. The cleavable linker permits a portion of the oligonucleotides in each population to be cleaved from the support to serve as templates for forming oligonucleotides whose sequences are perfectly complementary to the tag complements remaining on the support. By cleaving a portion of the tag complements from the one or more supports, and using the cleaved tag complements as templates to form complementary tags, the method produces matched tag and tag complement repertoires in greater yield and fidelity than previous methods. The invention therefore enhances hybridization specificity and tag sequence complexity relative to what is achieved when tags and their complements are synthesized independently.

The invention provides a substantial improvement in techniques that utilize oligonucleotide tags, by providing a method for enhancing the fidelity of synthesis of tag complements. Previously, tags were synthesized independently from tag complements, such that there was no assurance that all intended tags and tag complements were actually synthesized. In particular, previous polynucleotide synthesis methods were susceptible to significant or complete failures in one or more nucleotide addition steps, such that resultant tags or tag complements did not have the intended sequences and cannot hybridize perfectly with their intended partners. Defective synthesis of oligonucleotides can prevent tagged sample species from locating and hybridizing to the intended tag complements, resulting in loss of signal and/or underestimation of the abundance of the affected sample species. In addition, the presence of defective tag sequences could lead to cross-hybridization with other tags, interfering with hybridization of those tags to their corresponding complements. Moreover, the complexity of the tag repertoires and tag complement repertoires was diminished.

These problems are overcome by the present invention, by using a selected portion of immobilized tag complement sequences as templates to form a repertoire of corresponding tags. Ligation of the tags to sample selected sample substances, preferably biomolecules such as polynucleotides or polypeptides, yields a mixture of tagged substances (e.g., sample polynucleotide fragments) which can be captured on an array of tag complements. The invention thus provides a way of ensuring that a tag repertoire, or a subset of a repertoire, can hybridize to a full range of tag complements.

### I. Definitions

"Tag" or "tag oligonucleotide" or "oligonucleotide tag" refers to an oligonucleotide that contains a nucleotide sequence capable of serving as an identifier label for an attached species, such as a sample oligonucleotide, and which is detectably distinguishable from other tags in a tag repertoire.

"Complement" or "tag complement" as used herein refers to an oligonucleotide to which an oligonucleotide tag specifically hybridizes to form a perfectly matched duplex or triplex. In embodiments where specific hybridization results in a triplex, the oligonucleotide tag may be selected to be either double-stranded or single-stranded. Thus, where triplexes are formed, the term "complement" is meant to encompass either a double stranded complement of a single stranded oligonucleotide tag or a single-stranded complement of a double-stranded oligonucleotide tag.

The term "oligonucleotide" as used herein means a linear oligomer of natural or modified nucleosidic monomers, including deoxyribonucleosides and ribonucleosides. Usually monomers are linked by phosphodiester bonds or analogs thereof (e.g., phosphorothioates, phosphoramidates, phosphonates, peptide nucleic acids (N-derivatized glycine polymers), and the like) to form oligonucleotides ranging in size from a few monomeric units, e.g. 3-4, to several tens of monomeric units, and up to 100 monomeric units. Whenever an oligonucleotide is represented by a sequence of letters, such as "ATGCCTG," it will be understood that the nucleotides are in 5'→3' order from left to right and that "A" denotes deoxyadenosine, "C" denotes deoxycytidine, "G" denotes deoxyguanosine, and "T" denotes thymidine, unless otherwise noted. Usually oligonucleotides of the invention comprise the four natural nucleotides. However, they may also comprise non-natural nucleotide analogs. It is clear to those skilled in the art when oligonucleotides having natural or non-natural nucleotides may be employed, e.g. where processing by enzymes is called for, usually oligonucleotides consisting of natural nucleotides are required.

"Polynucleotide" or "polynucleotide fragment" refers to a polymer containing two or more nucleotides. "Polynucleotide" encompasses "oligonucleotide", although the length of a polynucleotide can exceed 100 monomeric units.

"Perfectly matched" in reference to a duplex means that the poly- or oligonucleotide strands making up the duplex form a double stranded structure with one other such that every nucleotide in each strand undergoes Watson-Crick basepairing with a nucleotide in the other strand. The term also encompasses the pairing of nucleoside analogs, such as deoxyinosine, nucleosides with 2-aminopurine bases, and the like, that may be employed. In reference to a triplex, the term means that the triplex consists of a perfectly matched duplex and a third strand in which every nucleotide undergoes Hoogsteen or reverse Hoogsteen association with a basepair of the perfectly matched duplex. Conversely, a "mismatch" in a duplex between a tag and an oligonucleotide means that a pair or triplet of nucleotides in the duplex or triplex fails to undergo Watson-Crick and/or Hoogsteen and/or reverse Hoogsteen bonding.

As used herein, "nucleoside" includes the natural nucleosides, including 2'-deoxy and 2'-hydroxyl forms, as described in Komberg and Baker, DNA Replication, 2nd Ed. (Freeman, San Francisco, 1992). "Analogs" in reference to nucleosides includes synthetic nucleosides having modified base moieties and/or modified sugar moieties, e.g. described by Scheit, Nucleotide Analogs (John Wiley, New York, 1980); Uhlman and Peyman, Chemical Reviews, 90: 543-584 (1990), or the like, with the only proviso that they are capable of specific hybridization. Such analogs include synthetic nucleosides designed to enhance binding properties, reduce complexity, increase specificity, and the like.

"Nucleotide" refers to a nucleoside or nucleoside analog having one or more phosphate groups (or analog thereof) attached to a 5', 3', and/or 2'-hydroxyl, or a nucleoside contained in an oligonucleotide or polynucleotide.

As used herein, "complexity" in reference to a population of polynucleotides means the number of different species of molecule present in the population.

As used herein, "repertoire" refers to a collection or mixture of difference-sequence oligonucleotides, e.g., comprising oligonucleotide tags or tag complements. A "repertoire" can have a defined complexity.

As used herein, "cleavable linking moiety" refers to a chemical group that is cleavable under selected chemical conditions. The term "cleavable linker" refers to a compound or chemical group that contains a cleavable linking moiety. For example, the term "cleavable linker" encompasses an activated form of a linker compound that can be coupled via a first linking functionality to a support, and via a second functionality to an oligonucleotide. The cleavable linker can contain a cleavable linking moiety, such as an ester group, which is cleavable under selected cleavage conditions.

### II. Components

The following sub-sections discuss various components, e.g., materials and reagents, that may be used to practice various aspects of the present invention.

### A. Solid Support

Solid phase supports for use with the invention can have any of a wide variety of forms, including microparticles, beads, membranes, slides, plates, micro-machined chips, and the like. Likewise, solid phase supports of the invention may comprise a wide variety of materials, including glass, plastic, silicon, alkanethiolate-derivatized gold, cellulose, low cross-linked and high cross-linked polystyrene, silica gel, polyamide, and the like. Preferably, either a population of discrete particles is employed, such that each particle has a uniform coating, or population, of complementary sequences for the same tag (and no other), or one or more supports are employed with spatially discrete regions, each region containing a uniform coating, or population, of sequences that are complementary to the same tag (and no other). In the latter embodiment, the area of the regions may vary according to particular applications. Usually, the regions range in area from several µm², e.g. 3-5 µm², to several hundred µm², e.g. 100-500 µm². Preferably, such regions are spatially discrete so that signals generated by events, e.g. fluorescent emissions, at adjacent regions can be resolved from each other by the detection system being employed. In some applications, it may be desirable to have regions with uniform coatings of more than one tag complement, e.g. for simultaneous sequence analysis, or for bringing separately tagged molecules into close proximity.

For example, a wide variety of microparticle supports may be used with the invention, including microparticles made of controlled pore glass (CPG), highly cross-linked polystyrene, acrylic copolymers, cellulose, nylon, dextran, latex, polyacrolein, and the like, disclosed in the following exemplary references: Meth. Enzymol., Section A, Vol. 44, pages 11-147, (Academic Press, New York, 1976); U.S. patents 4,678,814; 4,413,070; and 4,046;720; and Pon, Chapter 19, in Agrawal, editor, Methods in Molecular Biology, Vol. 20, (Humana Press, Totowa, NJ, 1993). Microparticle supports further include commercially available nucleoside-derivatized CPG and polystyrene beads (e.g. available from Perkin-Elmer Applied Biosystems, Foster City, CA); derivatized magnetic beads; polystyrene grafted with polyethylene glycol (e.g., TentaGel™, Rapp Polymere, Tubingen, Germany); and the like. Selection of the support characteristics, such as material, porosity, size, shape, and the like, and the type of linking moiety employed will depend on the conditions under which the tags are used. For example, in applications involving successive processing with enzymes, supports and linkers that minimize steric hindrance of the enzymes and that facilitate access to substrate are preferred. Other important factors to be considered in selecting the most appropriate microparticle support include size uniformity, efficiency as a synthesis support, surface area, and optical properties. It is noted that smooth, clear (transparent) beads provide instrumentational advantages when handling large numbers of beads on a surface.

As mentioned above, tag complements may also be synthesized on one or more solid phase supports to form an array of regions uniformly coated with tag complements. That is, within each region in such an array the same tag complement is synthesized. Techniques for synthesizing such arrays are now well known and are disclosed, for example, in McGall et al. (WO 93/22680 (Affymax); Pease et al., Proc. Natl. Acad. Sci. 91:5022-5026 (1994); Southern and Maskos (WO 90/03382 by Isis); Southern et al., Genomics, 13: 1008-1017 (1992); and Maskos and Southern, Nucleic Acids Research, 21:4663-4669 (1993).

Preferably, the invention is implemented with microparticles or beads uniformly coated with complements of the same tag sequence. Microparticle supports and methods of covalently or noncovalently linking oligonucleotides to their surfaces are well known, as exemplified by the following references: Beaucage and Iyer (cited above); Gait, editor, Oligonucleotide Synthesis: A Practical Approach (IRL Press, Oxford, 1984 and 1990 editions); and the references cited above. Generally, the size and shape of a microparticle is not critical; however, microparticles in the size range of a few, e.g. 1-2, to several hundred, e.g. 200-1000 µm diameter are preferable, as they facilitate the construction and manipulation of large repertoires of oligonucleotide tags with minimal reagent and sample usage.

In some preferred applications, commercially available controlled-pore glass (CPG) or polystyrene supports are employed as solid phase supports in the invention. Such supports come available with base-labile linkers and initial nucleosides attached, e.g. Applied Biosystems (Foster City, CA). Preferably, microparticles having pore size between 500 and 1000 angstroms are employed.

In other preferred applications, non-porous microparticles are employed for their optical properties, which may be advantageously used when tracking large numbers of microparticles on planar supports, such as a microscope slide. Particularly preferred non-porous microparticles are the glycidal methacrylate (GMA) beads available from Bangs Laboratories (Carmel, IN). Such microparticles are useful in a variety of sizes and derivatized with a variety of linkage groups for synthesizing tags or tag complements. Preferably, for massively parallel manipulations of tagged microparticles, GMA beads having diameters of about 5 µm diameter are employed.

### B. Tag Sequences

The tags of the invention are designed to provide unique tagging sequences for sample species of interest, and for specific hybridization to corresponding tag complements immobilized on one or more solid supports. The sequences of the tags (and tag complements) can be any group of sequences selected by the user, provided that (1) each different tag and tag complement does not significantly cross-hybridize with any other tag or tag complement in the repertoire, under selective hybridization conditions for sorting, (2) each tag and tag complement does not form secondary structure that would prevent specific hybridization with the intended complementary sequence, and (3) each tag can specifically recognizes and hybridize to its corresponding tag complement under the same conditions for all tags in the selected tag repertoire. Generally, the number of different tags increases in proportion to the number of different sample species that are to be processed in parallel. Tag sequences may be designed in accordance with any suitable method, such that the desired sequence specificity and sensitivity is achieved.

In a preferred embodiment of the invention, the oligonucleotide tag sequences are selected from a "minimally cross-hybridizing set" of oligonucleotide sequences. Preferably, the sequences of any two oligonucleotide tags of a tag repertoire differ by at least two nucleotides when the oligonucleotides are aligned to achieve maximum sequence identity. In other words, duplex or triplex formation between any tag in a repertoire and any other tag in the repertoire, or a tag complement thereof, results in at least two mismatches. In a more particular embodiment, the tag sequences in a tag repertoire are designed to ensure that duplexes and triplexes cannot form with any other tags or tag complements in the repertoire without forming at least three mismatched nucleotides, and so on. In such embodiments, greater specificity is achieved as the minimum number of mismatches increases, but the total repertoire of tags is smaller. Thus, for tags of a given length, there is a trade-off between the desired specificity and the size of repertoire.

The nucleotide sequences for oligonucleotides of a minimally cross-hybridizing set (repertoire) can be conveniently enumerated by simple computer programs following the procedures described in PCT Publication WO 96/41011 (see especially Fig. 1, the programs provided in Appendices Ia and Ib, and the corresponding discussion in the text) and PCT Publication WO 97/46704 (Appendices I and II). Program minhx of Appendix Ia in WO 96/41011 computes all minimally cross-hybridizing sets having 4-mer subunits composed of three kinds of nucleotides. Program tagN of Appendix Ib (WO 96/41011) enumerates longer oligonucleotides of a minimally cross-hybridizing set. Program 3tagN (WO 97/46704 at Appendix II) can be used to produce minimally cross-hybridizing sets of double stranded tags for binding single-stranded tag complements. Similar algorithms and computer programs are readily written for listing oligonucleotides of minimally cross-hybridizing sets for any embodiment of the invention. Table I below provides guidance as to the size of sets of minimally cross-hybridizing oligonucleotides for the indicated lengths and number of nucleotide differences, wherein the above-mentioned computer programs were used to generate the numbers shown.

**Table I**

| Oligonucleotide Subunit Length | Nucleotide Difference between Oligonucleotides of Minimally Cross-Hybridizing Set | Maximal Size of Minimally Cross-Hybridizing Set | Size of Repertoire with Four Subunits | Size of Repertoire with Five Subunits |
|---|---|---|---|---|
| 4 | 3 | 9 | 6561 | 5.90 x 10⁴ |
| 6 | 3 | 27 | 5.3 x 10⁵ | 1.43 x 10⁷ |
| 7 | 4 | 27 | 5.3 x 10⁵ | 1.43 x 10⁷ |
| 7 | 5 | 8 | 4096 | 3.28 x 10⁴ |
| 8 | 3 | 190 | 1.30 x 10⁹ | 2.48 x 10¹¹ |
| 8 | 4 | 62 | 1.48 x 10⁷ | 9.16 x 10⁸ |
| 8 | 5 | 18 | 1.05 x 10⁵ | 1.89 x 10⁶ |
| 9 | 5 | 39 | 2.31 x 10⁶ | 9.02 x 10⁷ |
| 10 | 5 | 332 | 1.21 x 10¹⁰ | |
| 10 | 6 | 28 | 6.15 x 10⁵ | 1.72 x 10⁷ |
| 11 | 5 | 187 | | |
| 18 | 6 | ≈25000 | | |
| 18 | 12 | 24 | | |

For some embodiments of the invention, where extremely large repertoires of tags are not required, oligonucleotide tags of a minimally cross-hybridizing set may be separately synthesized. Sets containing several hundred to several thousands, or even several tens of thousands, of oligonucleotides may be synthesized directly by a variety of parallel synthesis approaches, e.g. as disclosed in Frank et al., U.S. patent 4,689,405; Frank et al., Nucleic Acids Research, 11: 4365-4377 (1983); Matson et al., Anal. Biochem., 224: 110-116 (1995); Fodor et al., International application PCT/US93/04145 (WO 93/22684); Pease et al., Proc. Natl. Acad. Sci., 91: 5022-5026 (1994); Southern et al., J. Biotechnology, 35: 217-227 (1994), Brennan (WO 94/27719); Lashkari et al., Proc. Natl. Acad. Sci., 92: 7912-7915 (1995); or the like.

Preferably, oligonucleotide tags of the invention are synthesized combinatorially out of subunits (also referred to as "words") that are three to nine, and preferably three to six, nucleotides in length and are selected from the same minimally cross-hybridizing set. For oligonucleotides in this range, the members of such sets may be enumerated by computer programs based on the algorithm of Fig. 1 from

Preferably, minimally cross-hybridizing sets comprise subunits that make approximately equivalent contributions to duplex (or triplex) stability as every other subunit in the set. In this way, the stability of perfectly matched duplexes between every subunit and its complement is approximately equal. Guidance for selecting such sets is provided by published techniques for selecting optimal PCR primers and calculating duplex stabilities, e.g. Rychlik et al., Nucleic Acids Research, 17: 8543-8551 (1989) and 18: 6409-6412 (1990); Breslauer et al., Proc. Natl. Acad. Sci., 83: 3746-3750 (1986); Wetmur, Crit. Rev. Biochem. Mol. Biol., 26: 227-259 (1991); and the like. For shorter tags, e.g. about 30 nucleotides or less, the algorithm described by Rychlik and Wetmur is preferred, and for longer tags, e.g. about 30-35 nucleotides or greater, an algorithm disclosed by Suggs et al., pages 683-693 in Brown, editor, ICN-UCLA Symp. Dev. Biol., Vol. 23 (Academic Press, New York, 1981) may be conveniently employed. Clearly, there are many approaches available to one skilled in the art for designing sets of minimally cross-hybridizing subunits within the scope of the invention. For example, to minimize the affects of different base-stacking energies of terminal nucleotides when subunits are assembled, subunits may be provided that have the same terminal nucleotides. In this way, when subunits are linked, the sum of the base-stacking energies of all the adjoining terminal nucleotides will be the same, thereby reducing or eliminating variability in tag melting temperatures.

A "word" of terminal nucleotides, shown in italics with underlining below, may also be added to each end of a tag so that a perfect match is always formed between it and a similar terminal "word" on any other tag complement. Such an augmented tag would have the form:

| | | | | |
|---|---|---|---|---|
| *W* | W₁ | W₂ ... Wₖ₋₁ | Wₖ | *W* |
| *W'* | W₁' | W₂' ... Wₖ₋₁' | Wₖ' | *W*' |

where the primed W's indicate complements. With ends of tags always forming perfectly matched duplexes, all mismatched subunits (words) will cause internal mismatches, thereby reducing the stability of tag-complement duplexes that otherwise would have mismatched words at their ends. It is well known that duplexes with internal mismatches are significantly less stable than duplexes with the same mismatch at a terminus.

A preferred embodiment of minimally cross-hybridizing sets are those whose words are made up of three of the four natural nucleotides. The absence of one type of nucleotide in the oligonucleotide tags permits the tags to be converted from double-to single-stranded form, if desired, using the 5'→3' exonuclease activity of a DNA polymerase to remove the tag complement strand (stripping reaction). The following is an exemplary minimally cross-hybridizing set of words each comprising four nucleotides selected from the group consisting of A, G, and T:

**Table II**

| Word: | w₁ | w₂ | w₃ | w₄ |
|---|---|---|---|---|
| Sequence: | GATT | TGAT | TAGA | TTTG |

| Word: | w₅ | w₆ | w₇ | w₈ |
|---|---|---|---|---|
| Sequence: | GTAA | AGTA | ATGT | AAAG |

In this set, each member would form a duplex having three mismatched bases with the complement of every other member.

Further exemplary minimally cross-hybridizing sets are listed below in Table III. Clearly, additional sets can be generated by substituting different groups of nucleotides, or by using subsets of known minimally cross-hybridizing sets.

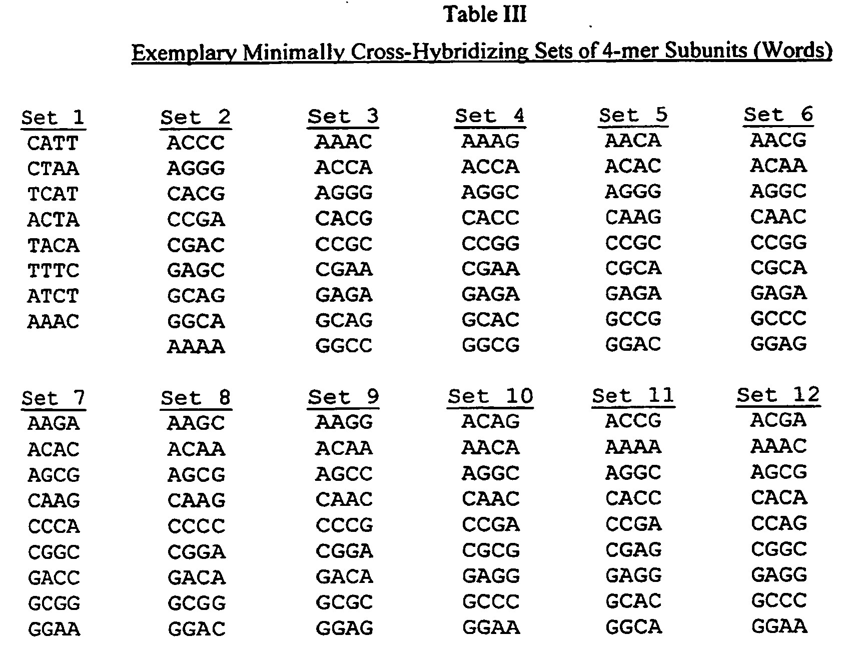

### C. Tag Synthesis

Oligonucleotide tag complements for use in the invention are conveniently synthesized on an automated DNA synthesizer, e.g. an Applied Biosystems model 392 or 394 DNA/RNA Synthesizer (Perkin-Elmer Applied Biosystems, Foster City, CA), or a "GENE ASSEMBLER PLUS" (Pharmacia), using standard chemistries, preferably phosphoramidite chemistry, e.g., as disclosed in the following references: Beaucage and Iyer, Tetrahedron, 48: 2223-2311 (1992); Molko et al., U.S. patent 4,980,460; Koster et al., U.S. patent 4,725,677; Caruthers et al., U.S. patents 4,415,732; 4,458,066; and 4,973,679; M.J. Gait (Ed.) in Oligonucleotide Synthesis, a Practical Approach, IRL Press, Oxford, England (1990); and the like.

When microparticles are used as supports, repertoires of tag complements may be generated by word-wise synthesis via "split and mix" techniques, e.g. as disclosed in Shortle et al. (WO 93/21203) or Lyttle et al., Biotechniques, 19: 274-280 (1995). Briefly, the basic unit of the synthesis is a subunit ("word") of the tag sequence. Preferably, phosphoramidite chemistry is used and 3' phosphoramidite oligonucleotides are prepared for each word in a minimally cross-hybridizing set, e.g. for the set first listed above, there would be eight 3'-phosphoramidite tetramers. Synthesis proceeds as disclosed by Shortle et al or in direct analogy with the techniques employed to generate diverse oligonucleotide libraries using nucleosidic monomers, e.g. as disclosed in Telenius et al., Genomics, 13: 718-725 (1992); Welsh et al., Nucleic Acids Research, 19: 5275-5279 (1991); Grothues et al., Nucleic Acids Research, 21: 1321-1322 (1993); Hartley, European patent application no. 90304496.4; Lam et al., Nature, 354: 82-84 (1991); Zuckerman et al., Int. J. Pept. Protein Research, 40: 498-507 (1992); and the like. Generally, these techniques simply call for the application of mixtures of the activated monomers (or polymer words) to the growing oligonucleotide during the coupling steps.

Preferably, tag complements are synthesized on a DNA synthesizer having a number of synthesis chambers which is greater than or equal to the number of different kinds of words used in the construction of the tags. That is, preferably there is a synthesis chamber corresponding to each type of word. In this embodiment, words are added nucleotide-by-nucleotide, such that if a word consists of five nucleotides there are five monomer couplings in each synthesis chamber. After a word is completely synthesized, the synthesis supports are removed from the chambers, mixed, and redistributed back to the chambers for the next cycle of word addition. This latter embodiment takes advantage of the high coupling yields of monomer addition, e.g. in phosphoramidite chemistries (e.g., see Example 1).

The tag and tag-complement sequences of the invention preferably have a length range from 12 to 60 nucleotides or basepairs. Preferably, the tag sequences range in length from 18 to 40 nucleotides or basepairs. More preferably, the tag sequences have lengths from 25 to 40 nucleotides or basepairs. In terms of preferred and more preferred numbers of words, these ranges may be expressed as follows:

**Table IV Numbers of Subunits (Words) in Preferred Tag Embodiments**

| Monomers in Subunit | Nucleotides in Oligonucleotide Tag | | |
|---|---|---|---|
| | (12-60) | (18-40) | (25-40) |
| 3 | 4-20 subunits | 6-13 subunits | 8-13 subunits |
| 4 | 3-15 subunits | 4-10 subunits | 6-10 subunits |
| 5 | 2-12 subunits | 3-8 subunits | 5-8 subunits |
| 6 | 2-10 subunits | 3-6 subunits | 4-6 subunits |

Most preferably, oligonucleotide tags are single-stranded, and specific hybridization occurs via Watson-Crick pairing with a tag complement.

Preferably, repertoires of tags and/or tag complements of the invention contain at least 100 members; more preferably at least 1000 members; and most preferably at least 10,000 members.

In embodiments where specific hybridization occurs via triplex formation, coding of tag sequences follows the same principles as for duplex-forming tags; however, there are further constraints on the selection of word sequences. Generally, third strand association via Hoogsteen type of binding is most stable along homopyrimidine-homopurine tracks in a double stranded target. Usually, base triplets form in T-A*T or C-G*C motifs (where "-" indicates Watson-Crick pairing and "*" indicates Hoogsteen type of binding); however, other motifs are also possible. For example, Hoogsteen base pairing permits parallel and antiparallel orientations between the third strand (the Hoogsteen strand) and the purine-rich strand of the duplex to which the third strand binds, depending on conditions and the composition of the strands. There is extensive guidance in the literature for selecting appropriate sequences, orientation, conditions, nucleoside type (e.g. whether ribose or deoxyribose nucleosides are employed), base modifications (e.g. methylated cytosine, and the like in order to maximize, or otherwise regulate, triplex stability as desired in particular embodiments, e.g. Roberts et al., Proc. Natl. Acad. Sci., 88: 9397-9401 (1991); Roberts et al., Science, 258: 1463-1466 (1992); Roberts et al., Proc. Natl. Acad. Sci., 93: 4320-4325 (1996); Distefano et al., Proc. Natl. Acad. Sci., 90: 1179-1183 (1993); Mergny et al., Biochemistry, 30: 9791-9798 (1991); Cheng et al., J. Am. Chem. Soc., 114: 4465-4474 (1992); Beal and Dervan, Nucleic Acids Research, 20: 2773-2776 (1992); Beal and Dervan, J. Am. Chem. Soc., 114: 4976-4982 (1992); Giovannangeli et al., Proc. Natl. Acad. Sci., 89: 8631-8635 (1992); Moser and Dervan, Science, 238: 645-650 (1987); McShan et al., J. Biol. Chem., 267:5712-5721 (1992); Yoon et al., Proc. Natl. Acad. Sci., 89: 3840-3844 (1992); Blume et al., Nucleic Acids Research, 20: 1777-1784 (1992); Thuong and Helene, Angew. Chem. Int. Ed. Engl. 32: 666-690 (1993); Escude et al., Proc. Natl. Acad. Sci., 93: 4365-4369 (1996); and the like.

Conditions for annealing single-stranded or duplex tags to their single-stranded or duplex complements are well known, e.g. Ji et al., Anal. Chem. 65: 1323-1328 (1993); Cantor et al., U.S. patent 5,482,836; and the like. Use of triplex tags has the advantage of not requiring a "stripping" reaction with polymerase to expose the tag for annealing to its complement, if the tag was initially prepared in double-stranded form.

Preferably, oligonucleotide tags of the invention employing triplex hybridization are double stranded DNA and the corresponding tag complements are single stranded. More preferably, 5-methylcytosine is used in place of cytosine in the tag complements in order to broaden the range of pH stability of the triplex formed between a tag and its complement. Preferred conditions for forming triplexes are fully disclosed in the above references. Briefly, hybridization takes place in concentrated salt solution, e.g. 1.0 M NaCl, 1.0 M potassium acetate, or the like, at pH below 5.5 (or 6.5 if 5-methylcytosine is employed). Hybridization temperature depends on the length and composition of the tag; however, for an 18-20-mer tag or longer, hybridization at room temperature is adequate. Washes may be conducted with less concentrated salt solutions, e.g. 10 mM sodium acetate, 100 mM MgCl₂, pH 5.8, at room temperature. Tags may be eluted from their tag complements by incubation in a similar salt solution at pH 9.0.

Minimally cross-hybridizing sets of oligonucleotide tags that form triplexes may be generated by the computer program 3tagN in Appendix II of WO 97/46704, or similar programs. An exemplary set of double stranded 8-mer words is given below in capital letters with the corresponding complements in small letters. Each such word differs from each of the other words in the set by three base pairs.

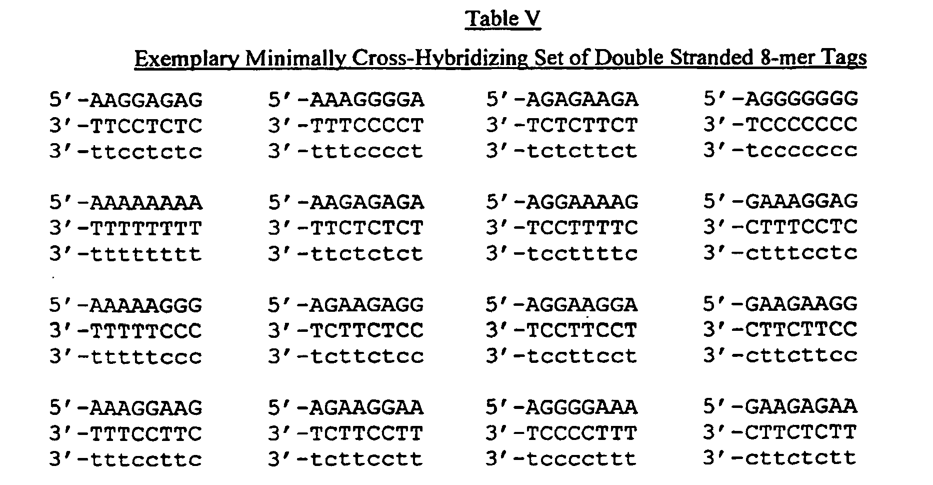

**TABLE VI Repertoire Size of Various Double Stranded Tags That Form Triplexes with Their Tag Complements**

| | | | | |
|---|---|---|---|---|
| Oligonucleotide Word Length | Nucleotide Diference between Oligonucleotides of Minimally Cross-Hibridizing Set | Maximal Size of Minimally Cross-Hibridizing Set | Size of repertoire with Four Words | Size of repertoire with Five Words |
| 4 | 2 | 8 | 4096 | 3.2 x 10⁴ |
| 6 | 3 | 8 | 4096 | 3.2 x 10⁴ |
| 8 | 3 | 16 | 6.5 x 10⁴ | 1.05 x 10⁶ |
| 10 | 5 | 8 | 4096 | |
| 15 | 5 | 92 | | |
| 20 | 6 | 765 | | |
| 20 | 8 | 92 | | |
| 20 | 10 | 22 | | |

Preferably, repertoires of double stranded oligonucleotide tags of the invention contain at least 10 members; more preferably, repertoires of such tags contain at least 100 members. Preferably, words are between 4 and 8 nucleotides in length for combinatorially synthesized double stranded oligonucleotide tags, and tag sequences for triplex formation are from 12 to 60 base pairs in length. More preferably, the tag sequences are from 18 to 40 base pairs in length.

### D. Solid Phase Linkers

During synthesis, tag complements of the invention are bound to one or more solid phase supports by first and second types of linking moieties, such that (1) both types of linking moieties remain intact (uncleaved) during tag complement synthesis, and (2) a first type of linking moiety can be cleaved to release the synthesized oligonucleotide under conditions in which the second linking moiety (or moieties) is/are not cleaved. Preferably, the cleavable linkage is a chemically cleavable linkage, i.e., suitable for selective cleavage without using a cleaving enzyme.

A large number of linking chemistries, and their cleavage properties, are known. Exemplary cleavable linking moieties, which are not intended to be limiting, include base-labile, acid-labile, photolabile, reducible, and enzyme-labile moieties.

Base-labile linkages include esters (-C(O)O-) thioesters (-C(O)S-), and 2-oxyethyl sulfones (-OCH₂CH₂-S(=O)₂R), for example. Such linkages can be cleaved under basic conditions, typically using a pH ≥ 8.0, preferably pH ≥ 9.0, for a suitable time, and optionally including a suitable nucleophile, such as ammonia, to increase pH and/or to promote cleavage by nucleophilic attack. For example, a di(2-oxyethyl)sulfone linking moiety can be cleaved by reaction with ammonia (28-30% in water) under mild conditions (55°C for 12-15 hours) without significant harm to the oligonucleotide (Example 1). Synthesis of a support containing a mixture of succinate ester (cleavable) and succinamide (non-cleavable) linking moieties is described in Example 3.

Acid-labile linkages include esters (-C(O)O-), thioesters (-C(O)S-), ketals (O-C(R₁R₂)-O-), hemiketals (O-CHR-O-), and phosphoramidates (-OP(=O)(O⁻)-N-) for example. For example, a phosphoramidate linkage can be introduced to a support by methods described in Hirschbein et al.

Pub. No. WO 97/31009) or Gryaznov et al. (U.S. Patent No. 5,599,922), and can be cleaved by treatment with 0.8% trifluoroacetic acid in dichloromethane for 40 minutes at room temperature (see also Gryaznov et al., Nucleic Acids Res. 20:3403-3409 (1992)).

Exemplary photolabile linkers include 2-nitrobenzyl esters, such as described by D.J. Yoo et al., Org. Chem. 60:3358-3364 (1995); D.L. McMinn et al., Tetrahedron 52:3827-3840 (1996); and U.S. Patent No. 5,430,136 to Urdea.

Reducible linkers are exemplified by disulfide linking groups which are readily reduced with 2-mercaptoethanol or dithiothreitol, for example (Gryaznov et al., Nucleic Acids Res. 21;1403-1408 (1993); U.S. Patent No. 5,118,605 to Urdea).

Enzyme-labile groups include any linking moiety that can be cleaved by an enzyme. Exemplary linking moieties include ester groups (-C(O)-O-), peptide groups (-NH-C(O)-), thioesters (-C(O)-S), and polynucleotide sequences that contain endonuclease restriction sites (e.g., U.S. Patent No. 4,775,619 to Urdea). In one preferred embodiment, the linking moiety is an oligopeptide containing an amino acid residue that is recognized by a specific protease, e.g., lysine-X or arginine-X (trypsin); aspartate-X (Staphylococcus aureus V8 protease); or tyrosine-X, tryptophan-X, phenylalanine-X, leucine-X, or methionine-X (α-chymotrypsin); where X represents the next residue in the N→C direction of the peptide. If cleavage of the oligopeptide linking group yields an oligonucleotide-peptide conjugate, the peptide portion can be removed, if desired, using pronase or other suitable protease, or the oligonucleotide can include an endonuclease restriction site that is positioned to permit selective removal of the peptide portion from the oligonucleotide.

Other chemical linkages are also contemplated, such as oligopeptides containing methionine, which are chemically cleavable using cyanogen bromide under acidic conditions. Additional references which describe cleavable linking moieties include Monforte et al. No. (WO 96/37630), Urdea (U.S. Patent 5,380,833), Wong, S.S., Chemistry of Protein Coniugation and Cross-Linking, CRC Press Boca Raton, FL, 1991 (particularly pp. 63-67) and Allen, G., Sequencing of Proteins and Peptides, Elsevier Science Pub. B.V., New York, 1983 (particularly Chapter 3).

Many stable linking moieties are also known that are generally resistant to the cleavage conditions mentioned above. Exemplary stable, "non-cleavable" moieties include ethers and polyethers, polyamines, phosphoramidites, phosphoramidates, and the like. These moieties are resistant under most acidic and basic conditions, in contrast to some of the groups noted above, and are therefore particularly convenient choices for the non-cleavable linkages used in the invention.

Exemplary linkers for attaching and/or synthesizing tags on microparticle surfaces are described in Pon et al., Biotechniques, 6:768-775 (1988); Webb, U.S. patent 4,659,774; Barany et al., (WO 92/04384); Brown et al., J. Chem. Soc. Commun., 1989: 891-893; Damha et al., Nucleic Acids Research, 18: 3813-3821 (1990); Beattie et al., Clin. Chem., 39: 719-722 (1993); Maskos and Southern, Nucleic Acids Research, 20: 1679-1684 (1992); and the like.

### III. Repertoire Synthesis Method

The present invention provides a method for synthesizing a repertoire of oligonucleotide tags and a corresponding repertoire of tag complements, such that synthesis fidelity and yields of desired tags are improved, and problems caused by failure sequences are reduced, relative to previous methods in which tags and tag complements were synthesized separately.

In one embodiment, the method entails (1) synthesizing a plurality of different-sequence oligonucleotide populations immobilized on one or more solid phase supports, wherein each population comprises a plurality of same-sequence oligonucleotides, and the oligonucleotides in each population comprise a tag complement sequence that is distinct relative to the tag complement sequences in the other populations, (2) cleaving a fraction of each oligonucleotide population from the one or more supports, and (3) inserting the cleaved oligonucleotides into a plurality of cloning vectors to form a tag-vector library. Such a process is illustrated in Fig. 2, where tag-vector construct 1, containing a cleaved tag complement sequence Tₙ, illustrates a member of a tag-vector library.

The solid phase support(s) for use in the invention can be prepared by any suitable method in light of the description in section II above. As noted above, the linking moieties that link the tag complements to the support comprise a mixture of at least two different linking moieties comprising a first, cleavable linking moiety and a second, non-cleavable linking moiety. Both the cleavable and non-cleavable linking moieties are selected to withstand the oligonucleotide synthesis protocol that is used to build the tag complement sequences on the support. The non-cleavable linking moiety must also be able to withstand conditions used later to hybridize tagged molecules to the support-bound tag complements.

The ratio of cleavable:non-cleavable linking moieties on the support is selected to ensure that (1) a sufficient amount of the tag complement repertoire synthesized on the support can be cleaved from the support, for preparing a complementary tag repertoire, (2) the cleaved oligonucleotides are clonable or can readily be made clonable, and (3) a sufficient amount of tag complements remains on the support for capturing tagged molecules when the support is used for sorting. Preferably, the cleavable linking moieties constitute less than 50%, and more preferably, from about 10% to about 30% of total bound tag complements. It is also preferred that a selected fraction of each population of synthesized oligonucleotides is bound to the support or supports via base-cleavable linkages.

A large number of suitably reactive supports are known, as discussed above, for attaching the desired linking moieties. For example, hydroxyl or amino-derivatized supports are commercially available or can be prepared from readily available materials, as discussed above, which are suitable for attaching selected linker groups.

The cleavable and non-cleavable linking groups can be attached to the support sequentially or simultaneously. Preferably, the linking moieties can be introduced onto a solid phase support by simultaneously reacting a mixture of cleavable and non-cleavable linkers with complementary reactive groups on the support. Thus, the relative proportions of cleavable and non-cleavable moieties on the support can be controlled by appropriate selection of the linker ratio for the derivatization reaction, taking into account the relative reactivities of the linkers with respect to the complementary reactive groups on the solid support. For example, when the cleavable and non-cleavable linkers are equally reactive toward the support, a cleavable:non-cleavable linker ratio of 10:90 can be used to produce a derivatized support in which 10% of the attached tag complements are cleavable from the support. Similarly, a ratio of 30:70 can be used to produce a derivatized support in which 30% of the tag complements are cleavable. If necessary, the relative proportion of a less reactive linker can be increased to offset a higher reactivity of the other linker-type.

For example, in the protocol described in Example 1, glycidal methacrylate/ethylene dimethacrylate (95/5) crosslinked beads derivatized with ethylene diamine were reacted simultaneously with a 10:90 molar ratio of cleavable:non-cleavable linkers, by standard phosphoramidite reaction chemistry. By this reaction, both linkers are attached to amino groups on the support to form phosphoramidite linkages which can later be oxidized to form stable phosphoramidate groups (Figure 1). The cleavable linker, which can be introduced on to the support as 2-[2-(4,4'-dimethoxytrityloxy)-ethylsulfonyl]ethyl-(2-cyanoethyl)-(N,N-diisopropyl)-phosphoramidite, contains a di(2-oxyethyl) sulfone moiety which is stable to acidic and oxidative conditions and remains intact during oligonucleotide synthesis, but is susceptible to base-catalyzed cleavage as shown in Figure 1. The non-cleavable linker in Fig. 1, which can be added to the support as 9-O-dimethoxytrityl-triethyleneglycol, 1-[(2-cyanoethyl)-N,N-diisopropyl)]-phosphor-amidite, comprises a polyethylene oxide linker that is stable to oligonucleotide synthesis conditions and to the basic conditions that can be used to cleave the cleavable linking moiety.

Oligonucleotides containing the tag complements are added to the linker-derivatized support using any appropriate reaction conditions. A wide variety of synthesis strategies have been developed in the art, and have been described (e.g., Gait, 1990 (supra), and other synthesis references cited above). Currently, the solid-phase phosphoramidite method is preferred, since it provides coupling yields of about 99% or greater. Preferably, synthesis is automated using any of a variety of available instruments and reagents to provide efficient synthesis.

To facilitate the preparation of a tag repertoire that is complementary to the tag complements, the immobilized tag complement oligonucleotides preferably include first and second primer segments that flank the tag complement sequence in each oligonucleotide. Thus, following attachment of the linkers to the support, a first primer sequence is preferably added to the linker, typically by sequential addition of the appropriate monomers to the linkers. In the protocol described in Example 1, a primer segment having the sequence 5'-TCCTTAATTAACTGGTCTCACTGTCGCA-3' (SEQ ID NO:1) is added by sequential monomer addition in the 3' to 5' direction using phosphoramidite-phosphotriester chemistry. Alternatively, a primer segment can be synthesized separately and can be attached to the linker in a single coupling reaction. Preferably, the linker and any attached primer sequence together provide a spacer moiety containing stretch of at least 10 chain atoms, to help separate the tag complement segment (discussed immediately below) from the surface of the support and from other tag complements on the support. Preferably, the spacer (including an optional primer-binding sequence) has a length of from 10 to 30 nucleotides. The presence of a spacer moiety is particularly useful to ensure that the tag complements will be readily accessible to hybridization with complementary tag sequences for sorting.

The tag complement sequence in each oligonucleotide can be added by sequential addition of monomers or blocks of monomers, or by simultaneous attachment of a separately synthesized repertoire of tag complements, with sequential addition of monomers being preferred. Repertoires can be formed on a planar array by photolithographic or robotic dispensing methods as are known in the art (supra). For repertoires formed on beads or particles, tag complement sequences are preferably formed combinatorially using a split and mix approach, such as described by Brenner in PCT Publication No. WO 96/41011. In brief, a plurality of different, minimally cross-hybridizing oligonucleotide "word" sequences are selected, which are designed to ensure that the synthesized tag complements will only be able to hybridize to their corresponding complementary tags under selected hybridization conditions, i.e., so that the levels of any incorrectly hybridized tags and tag complements are insignificant.

In the protocol described in Example 1, each tag complement sequence consists of a linear string of eight "words", each selected from the same set of eight tetrameric words. The primer-derivatized beads are divided into eight aliquots which are loaded into eight separate synthesis columns on an automated synthesizer. In each column, a different word selected from the eight possible words is added to the beads by sequential addition of appropriate monomers. After synthesis of the first word in each column is complete, the beads from the eight columns are combined and gently mixed together to homogeneity. After mixing, the bead mixture is divided again into equal aliquots which are loaded into the eight columns for another cycle of word addition. A total of eight cycles of word addition are performed, to produce beads with a total of 8⁸ (≅ 1.7 x 10⁷) different possible tag complement sequences. The split and mix protocol ensures that each bead contains a substantially uniform population of same-sequence oligonucleotides, i.e., the oligonucleotides on a particular bead have substantially the same sequences.

If necessary, the sequences of immobilized tag complements can be determined directly on the solid phase support by any known sequencing method. Preferably, the tag complements contain a universal primer-binding sequence on the 3'-side of the tag complement sequence, so that the tag complements can be sequenced by the Sanger dideoxy sequencing method, by annealing and extending a complementary sequencing primer. However, usually it is not necessary to know the sequences of the immobilized tag complements.

Following addition of the tag complement sequence, additional nucleotide residues can be appended to incorporate a second primer sequence, one or more endonuclease restriction sites, and/or any other desired features. For example, in the protocol of Example 1, a hexameric block of GGGCCC is appended to the tag complement to (1) help anchor the tag complement to its corresponding complementary tag during hybridization, (2) define the end of the tag complement segment, and (3) introduce a Bsp120I restriction site for subsequent ligation to a vector. This is followed by addition of a second primer sequence (primer 2) for optional amplification as discussed further below.

Thus, in a preferred embodiment, the immobilized oligonucleotides that contain the tag complement sequences include the following features:
[support] - [linking moiety] - [spacer] - [tag complement] - [terminal segment]

wherein the linking moiety is either cleavable or non-cleavable as discussed above, the spacer segment (preferably having a length of 10 to 30 nucleotides) optionally contains (1) an endonuclease restriction site and/or (2) a universal primer-binding sequence, the tag complement segment (preferably having a length of 12 to 60, 18 to 40, or 25 to 40 nucleotides) has the features discussed above, and the terminal segment (preferably having a length of 0-40 nucleotides, or 10 to 30 nucleotides) optionally contains (1) an endonuclease restriction site which may be the same or different from the first restriction site and (2) a universal primer sequence which may be the same or different from the complement of any primer-binding sequence in the spacer. In one preferred embodiment, the tag complement sequence in each oligonucleotide is flanked by first and second endonuclease restriction sites which may be the same or different, to facilitate subsequent ligation into a selected vector.

After oligonucleotide synthesis is complete, the oligonucleotides are deprotected if necessary, and a portion of the tag complement repertoire is cleaved from the support(s) using appropriate reaction conditions to selectively cleave only the cleavable linking moeities, while leaving the non-cleavable linking moieties intact.

Conveniently, the cleavage step and any deprotection steps are performed simultaneously using a single reagent. For example, if the cleavable linking moiety and one or more protecting groups are base-labile, then the support can be treated with base to remove the protecting groups and cleave the cleavable oligonucleotides from the support. Of course, the deprotection and cleavage steps can also be performed separately.

The cleaved oligonucleotide mixture can be inserted directly into a selected cloning vector, as discussed further below. However, the mixture is preferably purified to remove failure sequences and other extraneous materials, using any convenient method. For example, the mixture can be rapidly and conveniently purified by HPLC by ion exchange or reverse-phase chromatography by known methods. Electrophoretic separations can also be used. For reverse-phase chromatography, the synthesized oligonucleotides preferably contain a hydrophobic group, such as trityl or dimethoxytrityl (DMT), to facilitate preferential adsorption oftritylated oligonucleotides on the adsorbent, while non-tritylated sequences (arising from failed coupling steps) are substantially unretained by the column. Typically, the desired oligonucleotide mixture elutes (or migrates) as a broad peak since a vast spectrum of sequences with different G/C content and distribution are present. The mixture may be further purified by ethanol extraction or the like, and the resultant mixture can be stored dry or in a stable storage medium, preferably below 0°C. The integrity of the sequences of the oligonucleotides can be evaluated by randomly sequencing individual members of the mixture (usually after the mixture has been inserted into a cloning vector).

For subsequent attachment to sample polynucleotides of interest, the cleaved oligonucleotides are preferably inserted into one or more cloning vectors to form a tag vector library. Any type of replicable vector can be used, such as a plasmid, phagemid, cosmid, or the like, which can be propagated in an appropriate host. The vector can be single-stranded or double-stranded. A variety of suitable vectors are available from commercial sources (e.g., New England Biolabs, Clontech, GibcoBRL, etc.) or have been described in the literature. The vector will generally contain one or more restriction sites for inserting foreign DNA, at least one selection marker (e.g., ampicillin resistance), optionally a universal primer sequence upstream of the insertion site (e.g., for sequencing the insert), and any necessary components for propagating the vector in an appropriate host. Preferably, the vector includes a first restriction site for inserting an oligonucleotide tag, and a second restriction site immediately adjacent the first site, for inserting a sample fragment in close proximity to a tag. Alternatively, the restriction site for inserting the sample fragment can be introduced into the vector via the tag oligonucleotide, which contains an endonuclease site immediately adjacent to the tag segment.

For insertion into a double-stranded vector, the cleaved oligonucleotides are preferably converted to double-stranded form by annealing a complementary primer to a universal 3'-primer segment in the oligonucleotides and extending the primer by any suitable means. The double stranded product contains a tag sequence that is perfectly base-paired with a corresponding tag complement sequence. Typically, the annealed primer is extended in the 3' direction using a DNA polymerase in the presence of a mixture of dNTPs. To increase the quantity of double stranded oligonucleotide for insertion into the vector, the oligonucleotide mixture may be amplified by polymerase chain reaction using a pair of primers that anneal to primer-binding sequences that flank the tag complement sequences, until the desired amount of double-stranded product is obtained.

The double-stranded tag oligonucleotide mixture can be inserted into a vector by standard ligation techniques, e.g., by blunt end or sticky end ligation. Typically, the vector and oligonucleotide mixture are each treated separately with one or more restriction enzymes to produce products that have cohesive ends. Preferably, the vector and oligo mixture are each treated separately with two different restriction enzymes to produce products having two different sticky ends, to help reduce religation of the vector without incorporating an insert. The cleaved vector can also be treated with calf intestinal phosphatase to inhibit self-ligation of vector without insert. For example, in Example 2D, the oligonucleotide mixture and vector are each treated with PacI and Bsp120I. The larger fragment from the cleaved vector is treated with calf intestinal phosphatase and then is reacted with the restricted oligonucleotide mixture in the presence of a ligase, to form a mixture of tag vectors.

The ligation product can then be used to transform a suitable host to create a tag-vector library. The transformants are cultured until the desired number of transformants have been collected. Generally, the number of transformants should exceed the complexity of the tag repertoire to ensure sampling of a high proportion of the members of the repertoire. Preferably, the amount of transformants are sufficient to ensure the presence of at least 90% of the repertoire in the tag-vector library, more preferably at least 95%, and more preferably greater than 99%.

Tag-vector libraries of the invention are useful for simultaneously and uniquely tagging large numbers of sample oligonucleotide fragments, so that the tagged oligonucleotides can be sorted onto an array of tag complements for further processing or analysis. Thus, the tag-vectors can be used to form a library of tagged polynucleotide fragments, by combining a tag-vector library with a mixture of different polynucleotide fragments under ligation conditions effective to insert the different fragments at a site adjacent to a tag-sequence in each vector, so that each sample fragment (Sₙ in Fig. 2) becomes associated with a different tag (Tₙ). One member of such a tag-vector library is represented by construct 2 in Fig. 2. Preferably, the complexity of the tag-vector library exceeds the complexity of the sample, to help reduce the possibility of the same tag becoming associated with two or more different sample fragments.

For example, in Example 2D, the tag-vector library pBP9 was digested with BbsI and BamHI to create a large vector fragment having a tag sequence adjacent to the BbsI site (within 20 nucleotides). The vector fragment was treated with phosphatase to remove 5'-phosphate groups, and an oligonucleotide sample mixture (cDNA) having BbsI/BamHI sticky ends was inserted by ligation into the vector. The ligation mixture was used to transform E. coli host cells, and aliquots comprising approximately 1.6 x 10⁵ clones were used to inoculate liquid cultures in order to expand the library. Plasmid DNA was extracted from these cultures and stored.

Tagged polynucleotides from the tagged-sample oligonucleotides in the library can be processed or analyzed by any desired method. To facilitate detection, the tagged polynucleotides can be amplified by PCR using a universal primer pair for co-amplification, followed by optional purification of the desired products from template and amplification reagents (Example 2E). If the tags are to be used in single-stranded form, for binding single or double-stranded tag complements, the tag strands can be separated from their complementary strands by denaturation and collection of the desired tag strands, e.g., by solid phase capture of biotinylated tag strands on a streptavidin support. Alternatively, the complementary strand can be removed using an exonuclease with 5'→3' or 3'→5' exonuclease activity in the presence of a selected dNTP that is not contained in the tag complement region, so that the exonuclease effectively stops at the end of the tag complement. For example, if the tag sequence contains A, C and T bases, but not G, then the complementary sequence contains T, G and A but not C. Use of an exonuclease in the presence of CTP is effective to remove the complementary sequence until the first C is reached in the complementary sequence.

The tagged oligonucleotides (also referred to as tagged oligonucleotide fragments) are hybridized to the immobilized tag complement repertoire under suitable hybridization conditions as discussed above or as illustrated in Example 2E. If the solid support comprises particles or beads, the particles or beads can be incubated in the presence of the tagged oligonucleotides with constant agitation to facilitate hybridization, followed by a washing step. If only a small proportion of the beads are hybridized with complementary tags, the hybridized beads can be separated from non-hybridized beads by any suitable method, e.g., by FACS (fluorescence activated cell sorting). For this purpose, the tagged polynucleotides preferably include a detectable label, such as a fluorescent label (e.g., via a PCR primer) to facilitate detection of hybridized tagged polynucleotides.

After hybridization to the immobilized tag complement repertoire, the tagged polynucleotides can be analyzed according to any suitable method. For example, immobilized tagged polynucleotides can be sequenced, e.g., using the immobilized polynucleotide as templates to generate labeled extension products by cycle sequencing, e.g., as taught by Brenner (WO 96/12039 WO 95/27080). Alternatively, hybridized polynucleotides can be denatured from the support and sequenced according to known solution phase methods.

The invention is also useful for sorting different cDNAs onto a solid support, for sequence analysis or quantification. For example, the tags can be used to conduct differential display experiments to characterize changes in transcript levels from different samples, e.g., for characterization of different cell and tissue-types, disease conditions, etc. The invention can also be used for determining the toxicity of various compounds as disclosed in WO 97/13877.

If the tagged molecules are polypeptides, the immobilized polypeptides can be further characterized, e.g., by treatment with target specific antibodies to identify specific polypeptide species. Synthesis of tag-peptide conjugates is described, for example, in WO 96/12014 at page 14.

Additional uses for the tag and tag-complement repertoires obtained by the method of the invention are disclosed, for example, in WO 95/27080; WO 96/12014; WO 96/12039; WO 96/13877; WO 96/41011; and WO 97146704 including: tracking, identifying, and/or sorting classes or subpopulations of molecules, DNA sequencing, mRNA fingerprinting, etc.

The invention may be further understood in light of the following examples, which are not intended in any way to limit the scope of the invention.

### Example 1

### Synthesis of Tag Complements

All commercial chemicals were of synthesis quality and were used without further purification. Oligonucleotide syntheses were performed using a GENE ASSEMBLER PLUS-SPECIAL /4 PRIMERS synthesizer (Pharmacia) using 5.6 µm amino-derivatized polymeric beads (95% glycidal methacrylate/5% ethylene dimethacrylate derivatized with ethylene diamine, from Bangs Laboratories, Inc., Fishers, Indiana). The beads were estimated to have an amino group density of about 10⁷ amino groups per bead. In addition to the 25 µm polypropylene filters supplied with the synthesis columns from Pharmacia, a GFA glass filter mat (Whatman) was added to the end of each synthesis column to improve retention of the beads. Alternatively, synthesis columns were constructed out of stainless steel tubing (1-5 mm length x 0.75 inch diameter) having screw-cap ends to hold a 2 µm pore size PEEK filter at each end to retain the beads (such a column with a 5 mm length has a capacity of about 600 mg of beads).

4.8 g of amino-derivatized beads were packed into each of 8 synthesis columns, each containing 600 mg of beads. To form the cleavable linker moieties, 250 mg of 5'-PHOSPHATE-ON reagent (2-{2-(4.4'-dimethoxytrityloxy)-ethylsulfonyl]ethyl-(2-cyano-ethyl)-(N,N-diisopropyl)-phosphoramidite, from Clontech, catalog no. 5210-2) was dissolved in 3.8 mL of anhydrous acetonitrile to give a concentration of 0.1 M. To form the non-cleavable linker moieties, 250 mg of SPACER PHOSPHORAMIDITE (9-O-dimethoxytrityl-triethyleneglycol, 1-[(2-cyanoethyl)-N,N-diisopropyl)]-phosphoramidite, from Clontech, catalog no. 5260-3) was separately dissolved in 3.8 mL anhydrous acetonitrile to give a final concentration of 0.1 M. These two solutions were combined in a ratio to provide any desired % cleavability. For example, to achieve 10% cleavability, 1 part PHOSPHATE-ON solution (e.g., 200 µL) was combined with 9 parts of SPACER PHOSPHORAMIDITE (e.g., 1800 µL) in a dry bottle, which was then attached to the extra phosphoramidite port on the GENE ASSEMBLER SPECIAL PRIMERS 4 instrument. All transfers were carried out with a syringe, and the bottles were blanketed with argon to minimize exposure to water vapor in air.

Following addition of the linkers (cleavable and non-cleavable) to the beads, the DNA sequence below (primer 1. SEQ ID NO:1) synthesized on the beads in the 3' to 5' direction using the phosphite triester (phosphoramidite) method (M.J. Gait, Editor, Oligonucleotide Synthesis, a Practical Approach, IRL Press, London, UK, 1990, particularly Chapter 3), using the following protected monomers: T (unprotected). A and C (benzoyl), and G (isobutyryl):

Next, a different "word" sequence was appended to the primer template sequence in each column by phosphoramidite synthesis, by sequential addition of the appropriate monomers. The sequences of the 8 words were:
1)
2)
3)
4)
5)
6)
7)
8)

Following completion of the first word addition step, the beads were collected from the columns, mixed together, and reloaded into the columns, after which each different "word" sequence was added separately again in the 8 columns by phosphoramidite synthesis as above, followed by bead collection, remixing, and column re-loading. This cycle of adding words and mixing/splitting beads was performed a total of 8 times to create an oligonucleotide library of about 1.7 x 10⁷ (= 8⁸) different oligonucleotide sequences. After this combinatorial synthesis was complete, the sequence 5'-CCC-3' (trityl-on) was added to the beads, resulting in a mixture of immobilized oligonucleotides having the following general formula (SEQ ID NO:2)

Following addition of the CCC segment, a portion of the beads (4.3 g = 90%) was deprotected with saturated aqueous ammonia (2 mL, 28-30% in water) at 55°C for 12 hours. The deprotected beads were then treated with acetic acid for 15 minutes to remove the terminal 5'-trityl group. After the acid was removed, the beads were washed with methanol and concentrated in vacuo. The beads were then ready for hybridization.

The remaining portion of the beads (0.5 g = 10%) were reacted with appropriate phosphoramidite monomers to add the sequence 5'-CCTTAGGG-3' (primer 2, with trityl on, SEQ ID NO:3) followed by deprotection with ammonia (2 mL) at 55°C for 12 hours. The ammonia treatment was also effective to cleave the cleavable linkers to release 10% of the oligonucleotides from the beads. The beads were removed by centrifugation, and the remaining ammonia solution was concentrated by vacuum to half of the original volume. The cleaved oligonucleotides in this mixture had the general formula (SEQ ID NO:4):

The oligonucleotide mixture was purified by HPLC chromatography using a 150 mm x 4.6 mm PLRP-S column from Polymer Laboratories (100 Angstrom pore size, 8 µm particles) on a Gilson HPLC instrument. A 2-stage solvent gradient was used: Buffer A: 0.2 M triethyl ammonium acetate and 2% acetonitrile in water; Buffer B: 100% acetonitrile; flow rate = 1 mL/min at room temperature; gradient program: 95% A for 1 minute; reduce to 80% A over 5 minutes and hold at 50% A for 5 minutes; return to 95% A over 5 minutes and maintain at 95% A for 10 minutes to re-equilibrate column. The entire, broad trityl-on peak was collected between 18 minutes and 20 minutes. The collected fractions were concentrated in vacuo, treated with glacial acetic acid for 15 minutes to remove the 5'-trityl groups, and concentrated in vacuo to dryness. The residue was dissolved in 300 µL water and transferred to a 1.5 mL microfuge tube. To this was added 100 µL of 4 M NaCl, and the mixture was vortexed. After addition I mL ethanol, the sample was vortexed and then chilled -20°C for 20 minutes. The precipitated DNA was centrifuged, and the supernatant was removed. The precipitation procedure was repeated twice more. The final purified mixture was designated BP9.

### Example 2

### Cloning and Preparation of Tags

A. Plasmid Cloning Vector. A plasmid cloning vector pLCV2 was created from plasmid vector pBC.SK' (Stratagene) as follows, using the following oligonucleotides:

Oligos S-723 and S-724 were kinased, annealed together, and ligated to pBC.SK⁻ which had been digested with KprI and XbaI and treated with calf intestinal alkaline phosphatase, to create plasmid pSW143.1.

Oligos S-785 and S-786 were kinased, annealed together, and ligated to plasmid pSWi43.1, which had been digested with XhoI and BamHI and treated with calf intestinal alkaline phosphatase, to create plasmid pSW 164.02.

Oligonucleotides S-960, S-961, S-962, and S-963 were kinased and annealed together to form a duplex consisting of the four oligonucleotides. Plasmid pSW164.02 was digested with XhoI and SapI. The digested DNA was electrophoresed in an agarose gel, and the approximately 3045 bp product was purified from the appropriate gel slice. Plasmid pUC4K (from Pharmacia) was digested with PstI and electrophoresed in an agarose gel. The approx. 1240 bp product was purified from the appropriate gel slice. The two plasmid products (from pSW164.02 and pUC4K) were ligated together with the S-960/961/962/963 duplex to create plasmid pLCV1.

DNA from Adenovirus5 (New England Biolabs) was digested with PacI and Bsp120I, treated with calf intestinal alkaline phosphatase, and electrophoresed in an agarose gel. The approx. 2853 bp product was purified from the appropriate gel slice. This fragment was ligated to plasmid pLCVI which had been digested with PacI and Bsp120I, to create plasmid pSW208.14.

Plasmid pSW208.14 was digested with XhoI, treated with calf intestinal alkaline phosphatase, and electrophoresed in an agarose gel. The approx. 5374 bp product was purified from the appropriate gel slice. This fragment was ligated to oligonucleotides S-1105 and S-1106 (which had been kinased and annealed together) to produce plasmid pLCV2, which contained the following elements (SEQ ID NO:15)

B. Construction of Tag Plasmid Library. A tag-plasmid library designated pBP9 was created as follows. A primer designated PEP-1 was synthesized having the sequence (Fam-TTP conjugate obtained from Perkin Elmer-Applied Biosystems Division). This PEP-1 primer was annealed to the combinatorial tag mixture from Example 1 (designated BP9), and the primer was extended by treatment with Sequenase DNA polymerase in the presence of the four standard dNTPs. The double-stranded product was purified, digested with PacI and Bsp120I, and ligated to the larger fragment produced from pLCV2 by digestion with PacI and Bsp1201 and removal of 5'-phosphate groups with calf intestinal alkaline phosphatase. The ligation product was electrophoresed in an agarose gel and purified from the gel slice using a QIAEX II kit (Qiagen). This ligated material was used to transform electrocompetent E. coli TOP10F' (Invitrogen) or XL1BlueMRF' (Stratagene). Ligation and transformation conditions were optimized by conducting preliminary ligation reactions using 1 µL vector fragment (~ 10 µg), and either no insert, 1 µL insert diluted 1:10, or 1 µL non-diluted insert (~1 pmol), using a RAPID LIGATION KIT (Boehringer Mannheim). After ligation, the ligation mixture was used to transform electrocompetent cells, and transformants were plated (100 µL neat, 100 µL 1:10 dilution, and 100 µL 1:100 dilution). Using this approach, the best vector/insert ratio was determined as the ratio that produced the highest number of colonies per initial DNA while maintaining a very low background. For library synthesis, the ligation conditions were scaled up as necessary. Exemplary conditions are as follows:

| | |
|---|---|
| 200 µL | vector (total obtained starting from 20 µg plasmid) |
| X µL | insert (PacI/Bsp120I digest) |
| 50 µL | 10X ligation buffer |
| 50 µL | 10 mM ATP |
| Y µL | water |
| 10 µL | T4 DNA ligase (2,000,000 U/mL) |

| | |
|---|---|
| → final volume = 500 µL | |

The ligation mixture was incubated overnight at 16°C, followed by extraction twice with one volume (500 µL) TE-saturated phenol/chloroform/isoamyl alcohol (25:24:1), once with chloroform, followed by ethanol precipitation. After centrifugation, washing, and drying, the pellet was resuspended in 50 µL water. Into each of several vials of electrocompetent cells (80-100 µL cells) were added 1 to 2 µL of ligation product. The following conditions were used for electroporation: 200 ohms, 25 µF, 1800 V in a chilled 0.1 cm electroporation cuvette. Immediately after electroporation, 1 mL SOC broth (Sambrook et al., supra., at Appendix A.2 under SOC Medium) was added (room temperature), and the mixture was transferred to a 15 mL Falcon tube and shaken at 220 rpm at 37°C for 1 hour. Each transformation mixture was then added to 50 mL prewarmed broth (TB or LB + chloramphenicol) in a shaking flask. To determine titers, small aliquots of each transformation (5 µL neat or diluted) were plated on LB agar plates containing 30 µg/mL chloramphenicol to determine titer, while the remainder was inoculated into a liquid culture and shaken in an incubator overnight. Transformations were repeated until the total number of independent transformants (clones) was estimated to be approximately 1.7 x 10⁸ based on dilution analyses. This amount of transformants is equal to 10-fold the amount of potential tag variants produced by random combinatorial linkage of eight different tetrameric "word' sequences to produce tag sequences having a total length of eight words (8⁸ = 1.7 x 10⁷). Plasmid DNA (pBP9) was extracted from the cultures and pooled. Note that cultures could be combined before plasmid isolation only if they had similar titers. Otherwise, plasmids were isolated separately and then mixed together proportionately to their titers. Approximately 1.5 mg pBP9 was obtained.

### C. Poly(A)⁺RNA Isolation

Poly(A)⁺RNA (approx. 30 µg from 5 x 10⁷ cells) was extracted from THP-1 cells using a FastTrack kit (Invitrogen) as recommended by the supplier. Poly(A)⁺RNA (approx. 15 µg from 5 x 10⁷ cells) was extracted from yeast cells using a STRAIGHT A's mRNA isolation system from Novagen. For cDNA synthesis, an ethanol suspension of polyA RNA was centrifuged in a cold microfuge at full speed for 30 minutes. The supernatant was removed and discarded, after which the pellet was recentrifuged briefly, and the supernatant was carefully removed by pipette. The sides of the tube were washed carefully with 0.75 mL of 70% ethanol, followed by centrifugation for 20 minutes. The supernatant was removed as before, to ensure that all liquid had been removed. The RNA pellet was resuspended in DEPC-treated water (32 µL was used for mRNA from 5 x 10⁷ mammalian cells; otherwise, the volume was adjusted accordingly). The resultant solution was spun to the bottom of the tube and placed on ice. RNA concentration was estimated by dilution in water, assuming 1.0 absorbance units at 260 nm (A260) = 40 µg/mL RNA (the A260/A280 ratio should be 2.0). Gloves were worn at all times to avoid nuclease contamination.

### D. cDNA Library Construction

First- and second-strand cDNA was synthesized using a cDNA Synthesis Kit (Stratagene) as recommended by the supplier, using RNAse-free tubes, except that the following custom primer was used for first strand synthesis (RT primer, SEQ ID NO: 17):
where "V" represents a mixture of A, C, and G. In brief, the following solutions were added to a sterile 0.5 mL tube:
2.25 µL 10X first strand buffer
1.35 µL first strand methyl nucleotide mixture
1.0 µL RT primer (50 pmol/µL)
n µL DEPC-treated water (n calculated as below)
0.45 µL RNase block ribonuclease inhibitor (40 U/µL)

The resultant mixture was flicked to mix the components and then was spun to the bottom of the tube. To the mixture was added 2.5 µg poly(A)⁺RNA from above (m µL), where m is the volume containing 2.5 µg RNA, and n (the volume of DEPC-treated water) was calculated to produce a final volume of 21.8 µL (n+m = 16.8 µL). To the mixture was added 0.7 µL MMLV reverse transcriptase (50 U/µL). The mixture was gently vortexed or flicked, and then was spun to the bottom of the tube. The mixture was incubated at 37°C for 60 minutes in a heat block or PCR machine, and then placed on ice.

For second strand cDNA synthesis, the first-strand cDNA product (22.5 µL) was placed on ice, and the following components were added in order:

| | |
|---|---|
| 100 µL | 10X second-strand buffer |
| 3 µL | second-strand nucleotide mixture |
| 57.0 µL | sterile water (not DEPC-treated) |
| 1.0 µL, | α-³²P-dATP (10 µCi/µL, not more than 2 weeks old) |

The mixture was flicked to mix the solution and then spun down briefly, followed by equilibration on ice for 5 minutes to reduce the number of hairpin structures. Next, the following enzymes were added:
1.0 µL RNAse H (1.5 U/µL)
5.5 µL DNA polymerase I (9.0 U/µL) The resultant solution was mixed and spun briefly, without allowing the temperature to exceed 16°C. The mixture was incubated at 16°C for 150 minutes (but not a longer time), after which it was placed on ice.

To the reaction tube was added 100 µL TE-saturated phenol/chloroform/isoamyl alcohol (25:24:1), and the mixture was vortexed thoroughly. The mixture was centrifuged for 5 minutes. The aqueous (upper) layer was carefully removed and transferred to a new tube without collecting any of the interface. 100 µL of chloroform :isoamyl alcohol (24:1) was added, and the mixture was vortexed thoroughly. The mixture was centrifuged for 5 minutes. The aqueous layer was collected and transferred to a new tube as before, and 1 µL of 5 M NaCl was added and vortexed.

The resultant double-stranded hemimethylated cDNA was size-fractionated on a Pharmacia SIZESEP 400 column (Cat. No. 27-5105). The column was equilibrated in STE buffer (1X STE = 100 mM NaCl, 20 mM Tris pH 7.5, 10 mM EDTA) by placing the SIZESEP gel in a spin column and allowing the buffer to drain out until the top of the gel bed was reached, followed by suspension of the gel bed in 2 mL TBE and allowing the buffer to drain to the top of the gel bed, and repeating the TBE suspension and draining steps two more times. The column was capped at both ends until use, to prevent drying. Immediately before use, the caps were removed, and the column was spun briefly at about 400 x g for 2 minutes to compact the gel and remove excess buffer. Next, the cDNA sample was applied carefully and slowly to the center of the flat surface at the top of the gel bed, so that the sample did not flow past the sides of the gel or through any cracks. The column was placed into a Falcon 2059 tube so that the lower tip of the column was placed over the top of a cap-less 1.5 mL microfuge tube to collect column effluent. The column was spun in the Falcon tube at about 400 x g for 2 minute, so that synthesis primer and small cDNAs were retained in the column, while cDNAs of the desired length collected in the microfuge tube. The collected cDNA mixture was transferred to a new, capped tube. To the collected cDNA was added (optionally) 1 µL PELLET PAINT (Novagen, Madison, WI), 0.1 volume of 3 M NaOAc, and 2.5 volumes EtOH. The resultant solution was stored at -70°C for at least 30 minutes or -20°C overnight. The double-stranded cDNA product had the following formula (SEQ ID NO: 18): where the X's indicate nucleotides in the cDNAs, V represents A, C or G, and B represents C, G or T. Note that the RT primer sequence was selected to give a BsmBI site in the cDNAs which results in a 5'-GCAT overhang upon digestion with BsmBI.

After spinning the ethanol/NaOAc/cDNA solution for 30 minutes in a cold microfuge and washing with 70% ethanol as before, the size-fractionated cDNA was digested to completion with DpnII by resuspending the cDNA pellet in a solution of 170 µL water, 20 µL 10X DpnII buffer, and 10 µL DpnII (10 U/µL), followed by incubation at 37°C for 2 hours.

The biotinylated fragments were purified using Dynal M-280 streptavidin beads. In preparation for biotin capture, the magnetic streptavidin beads were resuspended by vortexing, and 100 µL bead (1 mg) was transferred to a fresh 1.5 mL microfuge tube. The tube was placed in a magnetic particle concentrator (MPC) for at least 60 seconds to allow the beads to pellet near the magnet. The supernatant was removed and discarded while the tube was in the MPC. To the tube was added 100 µL 1X B&W buffer (2X B&W buffer = 20 mM Tris-HCl pH 8.0,2 mM EDTA, and 2 M NaCl), and the suspension was vortexed, placed in the MPC to concentrate the magnetic beads, and the supernatant was removed as before. This washing procedure was repeated twice more. At no time were the beads allowed to dry during the above procedure.

To the DpnII digest from above (200 µL) was added 200 µL of 2X B&W buffer. This mixture was transferred to a tube containing the prepared magnetic streptavidin beads, and the beads were resuspended by flicking the tube. The tube was incubated in a Thermomixer at 25°C for 30 minutes so that the beads were fully suspended at all times to help ensure biotin capture. Next, the tube was placed in the MPC, and the supernatant was withdrawn and stored at -20°C. The beads were washed five times with 400 µL X B&W buffer, and the supernatants were analyzed to verify that little or no radioactive counts were present after the third or fourth wash.

To remove the tagged cDNA fragments, the following steps were performed::
1. Resuspend beads in a mixture of 20 µL, 10X NEB 3 Buffer (New England Biolabs) and 168 µL water.
2. Add 2 µL bovine serum albumin 100X stock (10 mg/mL BSA, New England Biolabs).
3. Add 10 µL BsmBI (4 U/µL).
4. Shake tube in Thermomixer at 55°C for 2 hours, so that beads remain fully suspended.
5. Add 7 µL BsmBI 10X stock solution, and shake tube at 55°C for 1-2 additional hours.
6. Place tube in MPC for 60 seconds. Collect supernatant, which contains released tag sequences.
7. Add 2 µL PELLET PAINT. Extract once with 200 µL phenol/chloroform and once with 200 µL chloroform.

To the resultant aqueous layer was added 20 µL 3 M NaOAc and 500 µL EtOH. This mixture was stored at -20°C overnight or at -70°C for 30 minutes. The solution was then spun in a microfuge for at least 30 minutes. The supernatant was removed and discarded, and the pellet was washed with, and centrifuged in, 0.5 mL of 70% EtOH. After the supernatant was removed, the cDNA pellet was resuspended in 10 µL water. The released cDNA fragment mixture had the following formula (SEQ ID NO:19):

The pBP9 tag-vector from above was digested with BbsI and BamHI, treated with calf intestinal alkaline phosphatase, and purified by electrophoresis in an agarose gel. The larger fragment (approximately 3.15 kb) was purified from the gel and combined with the cDNA fragment mixture for ligation. Note that the vector has been engineered so that BbsI digestion produces an end compatible with the BsmBI-digested end of the cDNAs. The purified vector fragment was ligated to the cDNA mixture prepared as above.

To produce a vector library of tagged cDNA inserts, the ligated vector-insert mixture was used to transform electrocompetent E. coli TOP 10 cells (Invitrogen). A small quantity of the transformation was plated on LB agar plates containing 30 µg/mL chloramphenicol to determine titer. Aliquots comprising approximately 1.6 x 10⁵ clones were used to inoculate liquid cultures in order to expand the library. Plasmid DNA was extracted from these cultures and stored.

### E. Hybridization of Tagged Fragments to Immobilized Tag Complements

The tagged DNA inserts can be captured or sorted by hybridization to an array of Immobilized tag complements such as described above.

Preferably, the tag cDNA conjugates are amplified from the vectors by PCR, using a conventional protocol such as the following. For each of 8 replicate PCRs, the following reaction components are combined: 1 µL vector DNA (125 ng/µL for a library, 10⁹ copies for a single clone); 14 µL 10X KLENTAQ Buffer (Clontech Labs, Palo Alto, CA); 0.25 µL biotinylated 20-mer "forward" PCR primer (1 nmol/µL); 0.25 µL FAM-labeled 20-mer "reverse" PCR primer (1nmol/µL); 1 µL 25 mM dATP, dGTP, dTTP, and 5-methyl-dCTP (total dNTP concentration 100 mM); 5 µL DMSO; 2 µL 50X KLENTAQ enzyme; and 80.5 µL water, for a total volume of 100 µL. The PCR reactions are run in an MJR DNA Engine (MJ Research), or like thermocycler, with the following protocol: (1) 94°C for 4 min; (2) 94°C for 30 sec; (3) 67°C for 3 min; (4) 8 cycles of steps 2 and 3; (5) 94°C for 30 sec; (6) 64°C for 3 min; (7) 22 cycles of steps 5 and 6; (8) 67°C for 3 min; and (9) hold at 4°C.

The 8 PCR mixtures are pooled and 700 µL phenol is added at room temperature, after which the combined mixture is vortexed for 20-30 sec and then centrifuged at high speed for 3 min (e.g., 14,000 rpm in an Eppendorf bench top centrifuge, or like instrument). The supernatant is removed and combined with 700 µL chloroform (24:1 mixture of chloroform:isoamyl alcohol) in a new tube, vortexed for 20-30 sec, and centrifuged for 1 min, after which the supernatant is transferred to a new tube and combined with 80 µL 3 M sodium acetate and 580 µL isopropanol. After centrifuging for 20 min, the supernatant is removed and 1 mL 70% ethanol is added. The mixture is centrifuged for 5-10 min, after which the ethanol is removed, and the precipitated DNA is dried in a microcentrifuge.

After resuspension, the cDNA is purified on avidinated magnetic beads (Dynal) using the manufacturer's recommended protocol. The cDNA is then digested with PacI (1 unit of enzyme per µg DNA), also using the manufacturer's recommended protocol (New England Biolabs, Beverly, MA). The cleaved DNA is extracted with phenol/chloroform followed by ethanol precipitation. The tags of the tag-cDNA conjugates are rendered single-stranded by adding 2 units of T4 DNA polymerase (New England Biolabs) per µg of streptavidin-purified DNA. 150 µg of streptavidin-purified DNA is resuspended in 200 µL water and combined with the following reaction components: 30 µL 10X NEB Buffer No. 2 (New England Biolabs); 9 µL 100 mM dGTP; 30 µL T4 DNA polymerase (10 U/µL); and 31 µL water; to give a final reaction volume of 300 µL. After incubation for 1 hour at 37°C, the reaction is stopped by adding 20 µL 0.5 M EDTA, and the T4 DNA polymerase is inactivated by incubating the reaction mixture for 20 min at 75°C. The tag-cDNA conjugates are purified by phenol/chloroform extraction and ethanol precipitation.

For hybridization of tag-cDNA conjugates to immobilized tag complements, the tag-cDNA conjugates prepared as above are suspended in 50 µL water, and the resulting mixture is combined with 40 µL 2.5X hybridization buffer. The combined mixture is filtered through a SPIN-X spin column (0.22 µm) using a conventional protocol to give a filtrate containing the tag-cDNA conjugates (5 mL of 2.5X hybridization buffer consists of 1.25 mL 0.1 M sodium phosphate, pH 7.2, 1.25 mL 5 M NaCl, 0.25 mL, 0.5% TWEEN 20, 1.5 mL 25% dextran sulfate, and 0.75 mL water). The number of beads in a given volume is readily estimated with a hemocytometer. Approximately 1.8 x 10⁷ beads derivatized with tag complements as above are dissolved in 10 µL TE/TWEEN buffer (TE with 0.1% TWEEN 20) and centrifuged so that the beads form a pellet, from which the TE/TWEEN is removed. To the beads, 25 µL of 1X hybridization buffer (10 mM sodium phosphate, pH 7.2, 500 mM NaCl, 0.01% TWEEN 20, and 3% dextran sulfate) is added and the mixture is vortexed to fully resuspend the beads, after which the mixture is centrifuged so that the beads form a pellet, and the supernatant is removed.

The tag-DNA conjugates from the above filtrate are incubated at 75°C for 3 min, and then are combined with the beads. The mixture is vortexed to fully resuspend the beads. The resulting mixture is further incubated at 75°C with vortexing for approximately three days (60 hours). After hybridization, the mixture is centrifuged for 2 min and the supernatant is removed. The beads are washed twice with 500 µL TE/TWEEN 20, and are resuspended in 500 µL 1X NEB buffer No. 2 with 0.1 % TWEEN 20. The beads are incubated at 64°C in this solution for 30 min, after which the mixture is centrifuged so that the beads form a pellet. The supernatant is removed, and the beads are resuspended in 500 µL TE/TWEEN. The hybridized conjugates may then be analyzed by any method known in the art or discussed herein.

### Example 3

### Succinate-Based Linking Moieties

The following procedure was used to make GMA beads derivatized with a cleavable succinate ester linking moiety (10%) and a non-cleavable succinate amide linking moiety (90%).

Succinate Ester. Succinic anhydride (240 mg, 2.4 mmol) was added in portions over 30 min to a stirred solution of 5'-O-dimethoxytrityl thymidine (3 mol) in anhydrous pyridine (6 mL) containing 4-dimethylaminopyridine (180 mg, 1.5 mmol). The reaction was stirred overnight and monitored by thin layer chromatography using chloroform:methanol 9:1 (v:v). The mixture was dried to a gum under reduced pressure. Residual pyridine was removed by co-evaporation with dry toluene (3 x 20 mL). The product was dissolved in dichloromethane (20 mL) and washed with ice-cold 10% aqueous citric acid (2 x 15 mL) and then with water (2 x 15 mL). The organic phase was dried over anhydrous sodium sulfate and evaporated under reduced pressure. The resultant foam was dissolved in dichloromethane (10 mL) and precipitated at room temperature into rapidly stirred hexane (250 mL). After the mixture was centrifuged, the supernatant was decanted. The precipitate was dried under reduced pressure to give a yield of 75-80% (ester product).

Succinamide. The above procedure for succinylation was repeated with an identical amount of 5'-O-dimethoxytrityl-3'-deoxy-3'-amino thymidine instead of 5'-O-dimethoxytrityl thymidine, to form the corresponding succinamide product (amide product).

Attachment to Support. 0.821 g (1.1 mmol) of the ester product or amide product was dissolved and stirred for 2 min in a mixture of 5 mL 0.2 M HOBt (1-hydroxybenzotriazole hydrate, 0.27 g in a mixture of 4.7 mL DMSO (dimethyl sulfoxide) and 4.7 mL NMP (N-methyl pyrrolidone)) and 5 mL of 0.2 M HBTU (O-benzotriazole-1-yl-N,N,N',N'-tetramethyluronium hexafluorophosphate, 1.03 g in a mixture of 1.4 mL N,N-diisopropylethylamine, 9.3 mL DMSO and 9.3 mL NMP). Amino-derivatized GMA beads (5.5g, 5.6 µm diameter) was stirred in 10 mL DMF. To this slurry was added all of the amide product from above and an amount of ester product equal to 1/9 of the amide product (9:1 molar ratio). The slurry was placed in a screw cap falcon tube and gently shaken for 2 hours. The mixture was then filtered and washed successively with 20 mL each of DMF, acetonitrile, methanol, and ether. The dried beads were then ready for synthesis of tag complements.

Although the invention has been described with respect to particular embodiments, it will be appreciated that various modifications can be made.

## Claims

1. A method of synthesizing a repertoire of oligonucleotide tags, the method comprising:
(a) synthesizing a plurality of different sequence oligonucleotide populations on one or more solid phase supports, such that each population is located at a spatially discrete region relative to the other populations, and the oligonucleotides in each population comprise a tag-complement sequence that is the same for every oligonucleotide in a given population;
(b) cleaving a fraction of the oligonucleotides from each population on the one or more supports, to release a mixture of different-sequence tag-complement oligonucleotides; and
(c) annealing a primer to each tag-complement oligonucleotide and extending each annealed primer to form a duplex comprising a tag-complement strand and a complementary tag strand, such that the tag-complement strands or the duplexes thus formed comprise an oligonucleotide tag repertoire.

2. The method of claim 1, wherein said oligonucleotides are bound to the one or more supports by their 3'-termini, and each oligonucleotide contains a universal primer-binding sequence on the 3'-side of the tag complement sequence.

3. The method of any of claims 1 to 2, wherein said fraction is from 10 to 30%.

4. The method of any of claims 1 to 3, wherein a selected fraction of each population of synthesized oligonucleotides is bound to the one or more supports via base-cleavable linkages, and said cleaving includes subjecting the immobilized oligonucleotides to basic conditions effective to cleave said fraction of oligonucleotides from the one or more supports.

5. The method of any of claims 1 to 4, wherein the tag complement sequence in each said oligonucleotide is flanked by first and second endonuclease restriction sites which may be the same or different.

6. The method of any of claims 1 to 5, wherein said repertoire of oligonucleotide tag complements contains at least 1000 different-sequence tag complements.

7. The method of claim 6, wherein said repertoire of oligonucleotide tag complements contains at least 10000 different-sequence tag complements.

8. The method of any of claims 1 to 7, wherein said tag complement sequences are from 12 to 60 nucleotides in length.

9. The method of claim 8, wherein said tag complement sequences are from 18 to 40 nucleotides in length.

10. The method of any of claims 1 to 9, wherein said one ore more solid phase supports are microparticles.

11. The method of claim 10, wherein said microparticle(s) have diameters of from 5 to about 40 *µ*m.

12. The method of any of claims 1 to 11, wherein said tag complement sequences each contain a plurality of segments that are (i) three to nine nucleotides in length and (ii) selected from a minimally cross-hybridizing set of tag complement sequences.

13. The method of claim 1 for use in forming a tag-vector library comprising multiple members from a tag repertoire, the method further comprising the step of
(d) inserting said duplexes by ligation into multiple copies of a selected vector, to form a tag-vector library comprising members of said tag-repertoire.

14. The method of claim 1 for use in forming a tag-vector library of tagged polynucleotide fragments, the method further comprising the steps of:
(d) inserting said duplexes by ligation into multiple copies of a selected vector, to form a tag-vector library comprising members of said tag-repertoire; and
(e) combining said tag-vector-library with a mixture of different polynucleotide fragments under ligation conditions effective to insert a polynucleotide fragment into each tag-vector at a site adjusted to a tag-sequence in each vector, thereby forming a library of tagged polynucleotide fragments.

15. A solid phase support comprising a population of oligonucleotides attached to a support, said population comprising first and second classes of oligonucleotides which contain identical oligonucleotide sequences, wherein the oligonucleotides in said first class contain a cleavable linking moiety that permits selective cleavage of the first class of oligonucleotides from the support, without significantly cleaving the second class of oligonucleotides.

16. The solid phase support of claim 15, wherein said support is a bead.

17. The solid phase support of claim 15, wherein said support is a region within a planar array of addressable regions, each region containing a plurality of immobilized oligonucleotides containing identical tag sequences, such that the tag sequences in at least two of the regions are different from each other.

18. A mixture of solid phase beads, said mixture comprising a plurality of beads, each having a population of oligonucleotides attached thereto, wherein each population comprises first and second classes of oligonucleotides such that the oligonucleotides in the first class contain a cleavable linking moiety that permits selective cleavage of the first class of oligonucleotides from the support, without significantly cleaving the second class of oligonucleotides, and the oligonucleotides in both classes in each population comprise a tag-complement sequence that is the same for every oligonucleotide in a given population.

## Patentansprüche

1. Verfahren zur Synthese eines Repertoires von Oligonucleotid-Anhängen(Tags), das Verfahren umfassend:
(a) Synthetisieren einer Vielzahl von unterschiedlichen Oligonucleotidsequenzpopulationen auf einem oder mehreren Festphasenträgern, in der Form, dass jede Population gegenüber den anderen Populationen in einem räumlich getrennten Bereich lokalisiert ist, und die Oligonucleotide in jeder Population eine Anhang(Tag)-Komplement-Sequenz umfassen, die für jedes Oligonucleotid in einer gegebenen Population die gleiche ist;
(b) Abspalten einer Fraktion der Oligonucleotide aus jeder Population auf dem einen oder mehreren Trägern, um ein Gemisch von unterschiedlichen Sequenz-Anhang(Tag)-Komplement-Oligonucleotiden freizusetzen; und
(c) Anlagern eines Primers an jedes Anhang(Tag)-Komplement-Oligonucleotid und Verlängern jedes angelagerten Primers, um ein Duplex umfassend einen Anhang(Tag)-Komplement-Strang und einen komplementären Anhang(Tag)-Strang zu bilden, in der Form, dass die so gebildeten Anhang(Tag)-Komplement-Stränge oder die Duplexe ein Oligonucleotid-Anhang(Tag)-Repertoire umfassen.

2. Verfahren nach Anspruch 1, wobei die Oligonucleotide über ihre 3'-Enden an den einen oder mehrere Träger gebunden sind, und jedes Oligonucleotid eine Universalprimerbindende Sequenz auf der 3'-Seite der Anhang(Tag)- Komplement-Sequenz enthält.

3. Verfahren nach einem der Ansprüche 1 oder 2, wobei die Fraktion 10% bis 30% beträgt.

4. Verfahren nach einem der Ansprüche 1 bis 3, wobei eine ausgewählte Fraktion von jeder Population synthetisierter Oligonucleotide über Basen-spaltbare Bindungen an den einen oder mehrere Träger gebunden ist, und das Abspalten den Schritt umfaßt, die immobilisierten Oligonucleotide basischen Bedingungen auszusetzen, die wirksam sind, um die Fraktion von Oligonucleotiden von dem einen oder mehreren Trägern abzuspalten.

5. Verfahren nach einem der Ansprüche 1 bis 4, wobei die Anhang(Tag)-Komplement-Sequenz in jedem Oligonucleotid von einer ersten und zweiten Restriktionsschnittstelle für eine Endonuclease flankiert ist, welche gleich oder unterschiedlich sein können.

6. Verfahren nach einem der Ansprüche 1 bis 5, wobei das Repertoire von Oligonucleotid-Anhang(Tag)-Komplementen mindestens 1000 unterschiedliche Sequenz-Anhang(Tag)-Komplemente enthält.

7. Verfahren nach Anspruch 6, wobei das Repertoire von Oligonucleotid-Anhang(Tag)-Komplementen mindestens 10000 unterschiedliche Sequenz-Anhang(Tag)-Komplemente enthält.

8. Verfahren nach einem der Ansprüche 1 bis 7, wobei die Anhang(Tag)-Komplement-Sequenzen eine Länge von 12 bis 60 Nucleotiden haben.

9. Verfahren nach Anspruch 8, wobei die Anhang(Tag)-Komplement-Sequenzen eine Länge von 18 bis 40 Nucleotiden haben.

10. Verfahren nach einem der Ansprüche 1 bis 9, wobei der eine oder mehrere Festphasenträger Mikropartikel sind.

11. Verfahren nach Anspruch 10, wobei das/die Mikropartikel Durchmesser von 5 bis etwa 40 µm hat/haben.

12. Verfahren nach einem der Ansprüche 1 bis 11, wobei jede der Anhang(Tag)-Komplement-Sequenzen eine Vielzahl von Segmenten enthält, die (i) eine Länge von 3 bis 9 Nucleotiden haben und (ii) von einem minimal kreuzhybridisierenden Satz von Anhang(Tag)-Komplement-Sequenzen ausgewählt sind.

13. Verfahren nach Anspruch 1 zur Verwendung bei der Erzeugung einer Anhang(Tag)-Vektorbank umfassend multiple Mitglieder eines Anhang(Tag)-Repertoires, wobei das Verfahren ferner den Schritt umfasst:
(d) Einfügen der Duplexe in multiple Kopien eines ausgewählten Vektors durch Ligierung, um eine Anhang(Tag)-Vektorbank umfassend die Mitglieder des Anhang(Tag)-Repertoires zu erzeugen.

14. Verfahren nach Anspruch 1 zur Verwendung bei der Bildung einer Anhang(Tag)-Vektorbank von angehängten Polynucleotidfragmenten, wobei das Verfahren ferner die Schritte umfasst:
(d) Einfügen der Duplexe in multiple Kopien eines ausgewählten Vektors durch Ligierung, um eine Anhang(Tag)-Vektorbank umfassend die Mitglieder des Anhang(Tag)-Repertoires zu erzeugen; und
(e) Kombinieren der Anhang(Tag)-Vektorbank mit einem Gemisch von unterschiedlichen Polynucleotidfragmenten unter Ligierungsbedingungen, die wirksam sind zur Insertion eines Polynucleotidfragments in jeden Anhang(Tag)-Vektor in eine Stelle, die an eine Anhang(Tag)-Sequenz in jedem Vektor angepasst ist, wobei eine Bank angehängter Polynucleotidfragmente erzeugt wird.

15. Festphasenträger, umfassend eine Population von Oligonucleotiden, die an einen Träger gebunden sind, wobei die Population eine erste und zweite Klasse von Oligonucleotiden umfasst, die identische Oligonucleotidsequenzen enthalten, wobei die Oligonucleotide in der ersten Klasse eine spaltbare Verknüpfungseinheit enthalten, die das selektive Abspalten der ersten Klasse von Oligonucleotiden vom Träger erlaubt, ohne die zweite Klasse von Oligonucleotiden signifikant abzuspalten.

16. Festphasenträger nach Anspruch 15, wobei der Träger eine Perle ist.

17. Festphasenträger nach Anspruch 15, wobei der Träger ein Bereich innerhalb einer planaren Anordnung mit adressierbaren Bereichen darstellt, wobei jeder Bereich eine Vielzahl von immobilisierten Oligonucleotiden mit identischen Anhang(Tag)-Sequenzen enthält, in der Form, dass die Anhang(Tag)-Sequenzen in mindestens zwei der Bereiche unterschiedlich voneinander sind.

18. Gemisch von Festphasenperlen, wobei das Gemisch eine Vielzahl von Perlen umfasst, jede mit einer Population von angehängten Oligonucleotiden, wobei jede Population eine erste und zweite Klasse von Oligonucleotiden umfaßt, in der Form, dass die Oligonucleotide in der ersten Klasse eine spaltbare Verknüpfungseinheit enthalten, die das selektive Abspalten der ersten Klasse von Oligonucleotiden vom Träger erlaubt, ohne die zweite Klasse von Oligonucleotiden signifikant abzuspalten, und die Oligonucleotide in beiden Klassen in jeder Population eine Anhang(Tag)-Komplement-Sequenz umfassen, die für jedes Oligonucleotid in einer gegebenen Population die gleiche ist.

## Revendications

1. Procédé de synthèse d'un répertoire de marqueurs oligonucléotidiques, le procédé comprenant :
(a) la synthèse d'une pluralité de populations oligonucléotidiques de séquences différentes sur un ou plusieurs supports en phase solide, de sorte que chaque population soit localisée dans une région spatiale discrète par rapport aux autres populations, et que les oligonucléotides dans chaque population comprennent une séquence complémentaire de marqueur qui est la même pour tous les oligonucléotides d'une population donnée ;
(b) le clivage d'une fraction des oligonucléotides de chaque population sur le un ou plusieurs supports pour libérer un mélange d'oligonucléotides complémentaires de marqueur avec différentes séquences; et
(c) l'hybridation d'une amorce sur chaque oligonucléotide complementaire de marqueur et l'extension de chaque amorce hybridée pour former un duplex comprenant un brin complémentaire de marqueur et un brin de marqueur complémentaire, de manière que les brins complémentaires de marqueur ou les duplex ainsi formés comprennent un répertoire de marqueurs oligonucléotidiques.

2. Procédé selon la revendication 1, dans lequel lesdits oligonucléotides sont liés au un ou plusieurs supports par leur extrémité 3', et chaque oligonucléotide contient une séquence de liaison à une amorce universelle sur le côté 3' de la séquence complémentaire de marqueur.

3. Procédé selon l'une quelconque des revendications 1 à 2, dans lequel ladite fraction est comprise entre 10 et 30%.

4. Procédé selon l'une quelconque des revendications 1 à 3, dans lequel une fraction choisie de chaque population d'oligonucléotides synthétisés est liée au un ou plusieurs supports par l'intermédiaire de liens clivables par une base, et ledit clivage comprend la soumission des oligonucléotides immobilisés à des conditions basiques efficaces pour cliver ladite fraction d'oligonucléotides à partir du un ou plusieurs supports.

5. Procédé selon l'une quelconque des revendications 1 à 4, dans lequel la séquence complémentaire de marqueur dans chaque dit oligonucléotide est flanquée par un premier et un second sites de restriction par endonucléase qui peuvent être identiques ou différents.

6. Procédé selon l'une quelconque des revendications 1 à 5, dans lequel ledit répertoire de complémentaires de marqueurs oligonucléotidiques contient au moins 1000 compléments de marqueurs de séquences différentes.

7. Procédé selon la revendication 6, dans lequel ledit répertoire de complémentaires de marqueurs oligonucléotidiques contient au moins 10000 compléments de marqueurs de séquences différentes.

8. Procédé selon l'une quelconque des revendications 1 à 7, dans lequel lesdites séquences complémentaires de marqueur ont une longueur comprise entre 12 et 60 nucléotides.

9. Procédé selon la revendication 8, dans lequel lesdites séquences complémentaires de marqueur ont une longueur comprise entre 18 et 40 nucléotides.

10. Procédé selon l'une quelconque des revendications 1 à 9, dans lequel ledit un ou plusieurs supports en phase solide sont des microparticules.

11. Procédé selon la revendication 10, dans lequel lesdites microparticules ont un diamètre compris entre 5 et environ 40 µm.

12. Procédé selon l'une quelconque des revendications 1 à 11, dans lequel les séquences complémentaires de marqueur contiennent chacune une pluralité de segments qui sont (i) d'une longueur comprise entre 3 et 9 nucléotides et (ii) sélectionnés à partir d'un ensemble de séquences complémentaires de marqueur présentant une hybridation croisée minimale.

13. Procédé selon la revendication 1 utilisé dans la constitution d'une banque de vecteurs marqueurs comprenant de multiples éléments d'un répertoire de marqueur, le procédé comprenant en outre l'étape
(d) d'insertion desdits duplex par ligature dans de multiples copies d'un vecteur sélectionné, pour constituer une banque de vecteurs marqueurs comprenant des éléments dudit répertoire de marqueurs.

14. Procédé selon la revendication 1 utilisé dans la constitution d'une banque de vecteurs marqueurs de fragments de polynucléotides marqués, le procédé comprenant en outre les étapes :
(d) d'insertion desdits duplex par ligature dans de multiples copies d'un vecteur sélectionné, pour constituer une banque de vecteurs marqueurs comprenant des éléments dudit répertoire de marqueurs ; et
(e) de combinaison de ladite banque de vecteurs marqueurs avec un mélange de différents fragments de polynucléotides dans des conditions de ligature efficaces pour l'insertion d'un fragment de polynucléotide dans chaque vecteur marqueur au niveau d'un site ajusté à la séquence marqueur de chaque vecteur, constituant ainsi une banque de fragments de polynucléotides marqués.

15. Support en phase solide comprenant une population d'oligonucléotides attachés à un support, ladite population comprenant une première et une seconde catégorie d'oligonucléotides qui comportent des séquences oligonucléotidiques identiques, dans lequel les oligonucléotides de ladite première catégorie comprennent un groupe de liaison clivable qui permet le clivage sélectif de la première catégorie d'oligonucléotides à partir du support, sans cliver significativement la seconde catégorie d'oligonucléotides.

16. Support en phase solide selon la revendication 15, dans lequel ledit support est une bille.

17. Support en phase solide selon la revendication 15, dans lequel ledit support est une région dans un réseau plan de régions identifiables, chaque région contenant plusieurs oligonucléotides immobilisés contenant des séquences de marqueurs identiques, de telle manière que les séquences de marqueurs dans au moins deux des régions soient différentes les unes des autres.

18. Mélange de billes de phase solide, ledit mélange comprenant une pluralité de billes, chacune ayant une population d'oligonucléotides attachée sur elle, dans lequel chaque population comprend une première et une seconde catégorie d'oligonucléotides telles que les oligonucléotides de la première catégorie contiennent un groupe de liaison clivable qui permet le clivage sélectif de la première catégorie d'oligonucléotides à partir du support, sans cliver significativement la seconde catégorie d'oligonucléotides, et les oligonucléotides des deux catégories dans chaque population comprennent une séquence complémentaire de marqueur qui est la même pour tous les oligonucléotides d'une population donnée.
